(19) Europäisches Patentamt
European Patent Office
Office européen des brevets

(11) Numéro de publication: **0 238 399 B1**

(12) **FASCICULE DE BREVET EUROPEEN**

(45) Date de publication de fascicule du brevet: **04.12.91**

(51) Int. Cl.5: **C07C 29/15**, C07C 31/04, C07C 31/08, C07C 31/10

(21) Numéro de dépôt: **87400546.5**

(22) Date de dépôt: **12.03.87**

(54) **Procédé de fabrication d'un mélange d'alcools primaires à partir de gaz de synthèse en présence d'un catalyseur contenant du cuivre, du cobalt, du zinc et au moins un métal alcalin et/ou alcalino-terreux.**

(30) Priorité: **17.03.86 FR 8603875**

(43) Date de publication de la demande: **23.09.87 Bulletin 87/39**

(45) Mention de la délivrance du brevet: **04.12.91 Bulletin 91/49**

(84) Etats contractants désignés: **BE DE FR GB IT NL SE**

(56) Documents cités:
**EP-A- 0 155 868**
**FR-A- 2 453 125**
**FR-A- 2 581 988**

(73) Titulaire: **INSTITUT FRANCAIS DU PETROLE**
**4, Avenue de Bois-Préau**
**F-92502 Rueil-Malmaison(FR)**

(72) Inventeur: **Courty, Philippe**
**91, rue Condorcet**
**F-78800 Houilles(FR)**
Inventeur: **Chaumette, Patrick**
**34, Côte de la Jonchère**
**F-78380 Bougival(FR)**
Inventeur: **Durand, Daniel**
**18, rue Michelet**
**F-92500 Rueil Malmaison(FR)**
Inventeur: **Verdon, Catherine**
**54, rue Eugène Labiche**
**F-92500 Rueil-Malmaison(FR)**

**EP 0 238 399 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à un procédé catalytique de fabrication d'un mélange de méthanol et d'alcools supérieurs par réaction d'oxyde de carbone avec l'hydrogène. Les alcools obtenus sont principalement des alcools primaires saturés. Le procédé de l'invention, permet d'obtenir une bonne sélectivité en alcools primaires linéaires et saturés en $C_2$ et plus.

On a décrit, dans les brevets US-A-4 122 110 et US-A-4 291 126 et dans les demandes de brevets FR-A-2 523 957 et FR-A-2 564 091 correspondantes aux demandes des Etats-Unis S.N. 478,764 déposée le 25 mars 1983 et S.N. 732,488 déposée le 10 mai 1985, l'utilisation de catalyseurs permettant de mettre en oeuvre un procédé de préparation d'un mélange d'alcools, à partir de mélanges CO, $H_2$ ou CO, $CO_2$, $H_2$. Ces catalyseurs présentent en général une bonne sélectivité dans la transformation des oxydes de carbone et de l'hydrogène en alcools; et leur sélectivité en alcools primaires linéaires et saturés en $C_2$ et plus, est souvent supérieure à 70% en poids. Enfin, leur productivité initiale est importante et le plus souvent supérieure ou égale à environ 0,1 tonne d'alcools par tonne de catalyseur et par heure.

Ces catalyseurs sont généralement constitués par au moins trois éléments essentiels: cuivre, cobalt et au moins un métal alcalin et/ou alcalino-terreux.

Les catalyseurs décrits dans le brevet US-A-4 122 110 contiennent en outre au moins un métal M choisi dans le groupe formé par le chrome, le fer, le vanadium et le manganèse ainsi qu'éventuellement du zinc et/ou de la magnésie et/ou un ciment alumineux.

Les catalyseurs décrits dans le brevet US-A-4 291126 contiennent, outre les éléments mentionnés pour les catalyseurs décrits dans US-A-4 122 110, au moins un métal du groupe des terres rares de numéros atomiques 57 à 71 inclus et éventuellement en outre au moins un métal noble du groupe VIII de la classification périodique des éléments (Handbook of Chemistry and Physics 37ème édition 1955-1956 p.392-393).

Les catalyseurs décrits dans la demande de brevet FR-A-2523957 contiennent,outre les éléments essentiels cités ci-dessus,de l'aluminium ainsi qu'éventuellement du zinc, au moins un métal M choisi dans le groupe formé par le manganèse, le vanadium, le fer et le rhénium, au moins un métal N choisi dans le groupe formé par le scandium, l'yttrium, le thorium, le zirconium et les métaux des terres rares de numéros atomiques 57 à 71 inclus, du chrome et au moins un métal noble du groupe VIII de la classification périodique des éléments.

Les catalyseurs décrits dans les documents mentionnés ci-dessus contiennent les éléments métalliques mentionnés dans des proportions pondérales et généralement des rapports atomiques bien déterminés.

Le demande de brevet européen EP-A-110357 décrit des catalyseurs contenant au moins quatre éléments dans leur formule tels que par exemple :

Cuivre, nickel, au moins un métal alcalin et/ou alcalinoterreux et au moins un métal des groupes $II_A$, $III_A$, $IV_A$, $II_B$, $III_B$, $IV_B$ et de la quatrième période des groupes $V_B$, $VI_B$ et $VII_B$ de la classification périodique des éléments ;

Ou zinc, au moins un composé choisi dans le groupe formé par le fer, le cobalt et le nickel, au moins un métal alcalin et/ou alcalino-terreux et au moins un métal des groupes $II_A$, $III_A$, $IV_A$, $II_B$, $III_B$, $IV_B$ et de la quatrième période des groupes $V_B$, $VI_B$ et $VII_B$ de la classification périodique des éléments.

Le brevet US-A-4440668 décrit des catalyseurs contenant essentiellement trois composés dérivés des métaux suivants :

1 -cuivre

2 -un métal choisi parmi les métaux des groupes $VI_B$, $VII_B$ et les métaux non nobles du groupe VIII.

3 -Un métal choisi parmi les métaux des groupes $IV_B$ et $V_B$.

Ces catalyseurs contiennent également de préférence 1 à 20% en poids d'au moins un métal alcalin.

La demande de brevet EP-A-100607 décrit des catalyseurs contenant au moins quatre éléments essentiels :

1- du cobalt

2- au moins un métal choisi dans le groupe formé par le cuivre, l'argent, le gallium, le zirconium, le zinc et le thorium

3- au moins un métal choisi dans le groupe formé par le palladium, le platine et le nickel

4- au moins un métal alcalin.

Les alcools obtenus par les procédés et en présence des catalyseurs précités présentent de nombreuses utilisations ; en particulier le fait d'obtenir une proportion importante d'alcools en $C_2$-$C_6$ est intéressant lorsque l'application envisagée est le mélange à des coupes d'hydrocarbures pour former des carburants mixtes hydrocarbures-alcools. Les alcools supérieurs ont en effet une meilleure compatibilité avec les hydrocarbures que le méthanol; ils facilitent également l'incorporation de ce même méthanol aux hydrocarbures.

Cependant, les procédés de synthèse d'alcools fondés sur l'utilisation des compositions catalytiques précités présentent en général divers inconvénients :

- Des quantités relativement importantes d'hydrocarbures sont produites parallèlement aux alcools.
- Lors de leur mise en contact avec le gaz de synthèse, les catalyseurs précités conduisent parfois à une réaction transitoire de méthanation, réaction fortement exothermique, qui nécessite d'opérer la substitution progressive du gaz inerte introduit dans l'unité avant admission du gaz de synthèse (après réduction par l'hydrogène et avant admission du gaz de synthèse) par le dit gaz de synthèse.
- En présence des éléments métalliques précités, la stabilisation thermique du couple cuivre-cobalt nécessite généralement l'addition de proportions importantes de métaux alcalins, ce qui implique généralement une détérioration de la pureté du mélange d'alcools produit.
- Pour ces diverses raisons, les procédures de conditionnement du catalyseur sous hydrogène et/ou sous gaz réactifs sont généralement complexes, (FR-A-2 523 957, et demande française déposée le 17 mai 1985 sous le numéro 8507581 correspondant à la demande des Etats-Unis d'Amérique S.N. 863,283 déposée le 15 mai 1986), ce qui complique la mise en oeuvre des catalyseurs précités.

Les nouveaux catalyseurs,décrits dans la présente invention, sont justiciables d'une procédure de conditionnement sous hydrogène et/ou sous gaz réactifs particulièrement simplifiée. De plus ces nouveaux catalyseurs présentent généralement une méthanation transitoire réduite, ce qui leur confère un avantage décisif aux plans de l'utilisation industrielle et de la sécurité d'exploitation.

Il a maintenant été trouvé que l'on peut obtenir des mélanges d'alcools particulièrement purs en utilisant des catalyseurs possédant une activité et une stabilité améliorées, dont la mise en oeuvre est plus simple que celle des catalyseurs de l'art antérieur et conduisant à des durées de vie particulièrement intéressantes.

Les catalyseurs utilisés selon l'invention dans la réaction de synthèse de mélange d'alcools à partir d'hydrogène et d'oxydes de carbone ($CO,CO_2$) sont formés essentiellement des éléments suivants : cuivre, cobalt, zinc, au moins un métal A choisi dans le groupe formé par les métaux alcalins et les métaux alcalino-terreux, et éventuellement le zirconium et/ou au moins un métal M et/ou au moins un métal N tels que définis ci-dessous.

Ces catalyseurs sont essentiellement exempt d'aluminium, de chrome, de fer, de vanadium et de manganèse, c'est-à-dire qu'ils ne contiennent pas ces métaux hormis à l'état d'impuretés,c'est-à-dire habituellement moins de 0,1% en poids, éventuellement apportées par les réactifs de départ et/ou les appareillages utilisés lors de leur préparation , et de préférence en quantité non décelable.

Dans les catalyseurs utilisés dans le procédé selon la présente invention, le métal M est choisi dans le groupe formé par le scandium, l'yttrium et les métaux des terres rares de numéros atomiques 57 à 71 inclus. Le métal M est de préférence choisi dans le groupe formé par le lanthane, le cérium, le praséodyme et le néodyme.

Dans les catalyseurs utilisés dans le procédé selon la présente invention, le métal N est choisi dans le groupe formé par les métaux nobles du groupe VIII (ruthénium, rhodium, palladium, osmium, iridium et platine). Le métal N est de préférence choisi dans le groupe formé par le rhodium, le palladium et le platine.

La proportion pondérale des différents métaux présente dans le catalyseur par rapport au poids total des métaux présents est donnée dans le tableau ci-après.

| métal | gamme large | gamme préférée | gamme la plus préférée |
|---|---|---|---|
| Cu | 15 - 55 | 20 - 45 | 20 - 45 |
| Co | 5 - 25 | 8 - 20 | 8 - 20 |
| Zn | 15 - 70 | 20 - 65 | 20 - 65 |
| Zr | 0 - 55 | 0 - 45 | 0 - 45 |
| Zn + Zr | 15 - 70 | 20 - 65 | 20 - 65 |
| métal A | 0,01 - 5 | 0,05 - 3,5 | 0,05 - 3,5 |
| métal M | 0 - 20 | 0 - 20 | 0,1 - 15 |
| métal N | 0 - 1 | 0 - 1 | 0,01 - 0,8 |

Au sein des catalyseurs de la présente invention les métaux M et/ou N peuvent être absents. Lorsque le métal N est absent et le métal M est présent, ce dernier sera de préférence en proportions pondérales telle que sa teneur soit de 0,1 à 15%, et de même si le métal M est absent et le métal N est présent, ce dernier sera de préférence en proportions pondérales telle que sa teneur soit de 0,01 à 0,8%.

Les métaux M et/ou N lorsqu'ils sont présents seront d'une manière la plus préférée en proportions

pondérales telles que la teneur en métal M soit de 5 à 15% en poids et celle en métal N de 0,02 à 0,8% en poids, les autres métaux restant à l'intérieur des gammes larges ou préférées mentionnées ci-dessus.

En outre, à l'intérieur des fourchettes de composition pondérales précitées, il est nécessaire que les différents métaux soit entre eux dans les proportions atomiques larges, préférées et les plus préférées données dans le tableau ci-après.

| proportions atomiques | larges | préférées | les plus préférées |
|---|---|---|---|
| Cu/Co | 0,2:1 à 5:1 | 0,5:1 à 3,5:1 | 1 :1 à 3:1 |
| (Zn + Zr)/Co | 0,5:1 à 8:1 | 1:1 à 5,5:1 | 1,2:1 à 5:1 |
| Zn/(Zn + Zr) | 0,05:1 à 1:1 | 0,1:1 à 1:1 | 0,3:1 à 1:1 |

Les formules catalytiques préférées sont les suivantes :

1. Cu + Co + Zn + $I_A$
2. Cu + Co + Zn + Zr + $I_A$
3. Cu + Co + Zn + VIII noble + $I_A$
4. Cu + Co + Zn + Zr + terres rares + $I_A$
5. Cu + Co + Zn + Zr + terres rares + VIII noble + $I_A$

Dans ces formules IA représente au moins un métal alcalin. Les formules catalytiques les plus préférées sont celles qui contiennent du zirconium.

La teneur en zirconium, dans ces formules catalytiques les plus préférées, est alors habituellement d'environ 1% à environ 45% en poids et de préférence d'environ 2% à environ 45% en poids ; la somme Zn + Zr et les autres métaux restant à l'intérieur des gammes larges, préférées ou les plus préférées mentionnées ci-avant.

Lorsque le zirconium est présent les proportions atomiques sont habituellement d'environ 1:1 à environ 5,5:1 et de préférence d'environ 2:1 à environ 5:1 pour le rapport (Zn + Zr)/Co et habituellement d'environ 0,1:1 à environ 0,98:1 et de préférence d'environ 0,3:1 à environ 0,98:1 pour le rapport Zn/(Zn + Zr).

Afin que les catalyseurs selon la présente invention soient à la fois actifs et stables dans la synthèse d'alcools supérieurs, comportant 2 atomes de carbone et plus dans leur molécule, et sélectifs dans la transformation de CO en composés oxygénés (les sous produits dont la formation doit être réduite au maximum sont les hydrocarbures), il est préférable qu'ils présentent une bonne homogénéité de composition et que les métaux les plus actifs, notamment le cobalt, soient uniformément répartis dans chaque particule élémentaire de catalyseur.

Les meilleurs résultats, en termes de sélectivité de transformation du CO en composés oxygénés et notamment en alcools supérieurs, sont obtenus avec des catalyseurs présentant une variation du rapport atomique zinc/cobalt et éventuellement zirconium/cobalt lorsque du zirconium est présent, inférieure à 15% et préférentiellement inférieure à 10% par rapport à la valeur moyenne de ce rapport, à l'échelle de 50 Å (5 nanomètres).

Pour obtenir des catalyseurs homogènes, il est important de préparer tout d'abord une solution (par définition, homogène) contenant le cuivre, le cobalt, le zinc et éventuellement le zirconium ainsi qu'éventuellement au moins un métal M et/ou éventuellement au moins un métal N puis de transformer cette solution par une réaction de complexation ou une réaction de coprécipitation en une substance solide, appelée le précurseur du catalyseur et présentant toujours une bonne homogénéité de composition.

Les métaux Cu, Co, Zn et éventuellement zirconium ainsi qu'éventuellement M et/ou N sont mis en oeuvre sous forme de composés solubles et de préférence solubles en milieu acide, bien que les complexes amminés (solubles en milieu ammoniacal) du cuivre, du cobalt, du zinc et de certains des métaux M et N puissent également être utilisés en addition au réactif alcalin et/ou ammoniacal de coprécipitation.

On utilisera par exemple les oxydes solubles, les hydroxydes, les carbonates, les hydroxycarbonates solubles en milieu acide (par exemple Cu $CO_3$ - $Cu(OH)_2$, $Co(OH)_2$, Zn $CO_3$-$Zn(OH)_2$,$Zr(OH)_4$, les nitrates, oxalates, tartrates, citrates, acétates, acétylacétonates ou encore des combinaisons anioniques telles que l'oxalatocobaltate, les zirconates solubles; les nitrates sont les sels solubles qui sont le plus souvent mis en oeuvre.

Le métal A peut être ajouté à toutes les étapes unitaires de la fabrication. On peut par exemple l'ajouter dans les solutions de départ, puis ajouter au moins un complexant, sécher et calciner; on peut également utiliser un carbonate, un bicarbonate, et/ou un hydroxyde d'au moins un métal A, préparer le précurseur du catalyseur par coprécipitation entre le composé du métal A et une solution contenant les autres métaux Cu Co Zn et éventuellement Zr et/ou M et/ou N et laisser dans le coprécipité une quantité contrôlée de métal A

par contrôle des lavages ultérieurs; mais il est parfois préférable, après coprécipitation et désalcalinisation par lavage soigneux de malaxer le précipité tel quel ou préalablement séché, avec une quantité contrôlée de solution de sels solubles du métal A; ou encore d'activer thermiquement le précipité des métaux Cu Co Zn et éventuellement Zr et/ou M et/ou N puis d'ajouter par malaxage comme précédemment au moins un métal A, alcalin et/ou alcalinoterreux.

Pour la préparation de ces masses catalytiques il est important d'utiliser des techniques de préparation permettant d'obtenir un produit de composition aussi homogène que possible, et évitant la ségrégation des différents éléments lors des différentes étapes unitaires de la préparation.

On décrit ci-après des méthodes préférées de préparation de masses catalytiques homogènes conduisant à des catalyseurs homogènes à la fois actifs et sélectifs pour la production d'alcools supérieurs et dont l'emploi conduit à une formation aussi faible que possible d'hydrocarbures. Ces méthodes permettent de maintenir l'homogénéité désirée au cours des étapes de la préparation.

Une méthode de préparation préférée qui a déjà été décrite dès 1968 par le demandeur dans les demandes de brevets français FR-A-1 604 707 et FR-A-2 045 612 consiste à préparer une solution contenant les métaux Cu Co Zn ainsi qu'éventuellement Zr et/ou M et/ou N et ainsi qu'éventuellement au moins un métal A puis à ajouter au moins un composé permettant la formation de complexes, choisi de préférence parmi :

- les acides organiques contenant deux ou plusieurs fonctions acides, par exemple, les acides oxalique, malonique, succinique ou glutarique,
- les acides alcools, par exemple les acides glycolique, lactique, malique, tartrique, ou de préférence citrique,
- les acides aminés, par exemple l'acide aminoacétique, l'alanine ou la leucine ; les alkanolamines par exemple monoéthanolamine, diéthanolamine, triéthanolamine dans une proportion d'environ 0,5 à 2 équivalent gramme $COO^-$ ou>-NH par équivalent gramme de métaux.

La solution obtenue est évaporée sous vide (par exemple dans un évaporateur rotatif) de manière à obtenir une solution d'une viscosité d'au moins 1 Pa.S qui est ensuite transférée dans une étuve opérant sous vide entre environ 60 et environ 120°C et séchée jusqu'à réduire le teneur en eau à moins de 10% poids. On obtient de la sorte une masse vitreuse transparente, homogène et amorphe en diffraction X qui est ensuite activée thermiquement sous azote ou en présence d'un gaz contenant de l'oxygène, par exemple entre environ 300 et environ 600°C pendant un temps suffisant pour ramener la teneur de l'oxyde en matières volatiles à moins de 10% et de préférence moins de 6% en poids.

Après activation thermique, on peut éventuellement ajouter au produit activé une quantité contrôlée de métal A selon le mode opératoire qui sera décrit ci-après.

Un autre mode de préparation préféré consiste à préparer, par au moins une réaction de coprécipitation, un précurseur hydraté, homogène, contenant les métaux Cu Co Zn et éventuellement Zr et/ou M et/ou N; la réaction de coprécipitation consiste à mettre en présence dans des conditions opératoires définies plus loin une solution de sels solubles des métaux Cu Co Zn, éventuellement Zr et/ou M et/ou N avec une solution de carbonate et/ou d'hydrogenocarbonate(bicarbonate) et/ou d'hydroxyde de métaux alcalins de préférence de sodium et/ou de potassium, et/ou d'ammonium de façon à obtenir un coprécipité qui, après lavage ultérieur, constitue le précurseur hydraté homogène.

Toutes les techniques et appareillages qui ont été décrits dans l'art antérieur peuvent être utilisés ou appliqués à la réalisation de l'invention; on peut par exemple ajouter la solution de sels des métaux Cu Co Zn et autres dans la solution alcaline ou l'inverse. De préférence, on ajoutera les deux solutions, de façon simultanée, et en régulant leurs débits par le pH mesuré dans la zone réactionnelle, dans un réacteur comportant un système d'agitation efficace. D'une façon préférée, les deux solutions seront mises en contact dans la zone de turbulence maximum délimitée par le volume enveloppant l'appareillage d'agitation à l'intérieur du volume réactionnel.

Le temps de séjour moyen, exprimé en minutes et défini comme le rapport du volume du réacteur exprimé en litres, au débit volumique total (litres/minute) des solutions injectées dans le dit réacteur, peut varier de 0,1 à 600 minutes, étant donné que la réaction peut être menée soit dans un réacteur opérant en continu (dit à concentration et autres conditions stationnaires), dont le volume utile peut varier de quelques $cm^3$ à environ 50 litres, où le temps de séjour varie de 0,1 à 15 minutes et où le produit de réaction est récupéré en continu, (éventuellement mûri dans un autre réacteur) puis envoyé par exemple sur un filtre presse ou encore sur un filtre rotatif où il est lavé; soit dans un réacteur opérant en "batch", où le temps de séjour est d'au moins 30 minutes et de préférence d'au moins 60 minutes, où les réactifs sont injectés en continu, sans récupération parallèle du produit de réaction, et où le produit de réaction reste en présence de réactifs injectés en continu. Ce type de réacteur dont le volume (compte tenu des spécifications de concentration des solutions utilisées et des quantités de catalyseur à préparer) varie environ de 1 litre à

environ 1000 litres ou plus, opère à concentrations variables, les autres conditions opératoires restant constantes au cours de la précipitation elle-même. Ce mode de réaction est plus adapté à la préparation de composés cristallisés, alors que le réacteur opérant en continu est plus adapté à la préparation de composés amorphes en diffraction X.

Un mode préféré de réalisation de l'invention consiste à faire réagir une solution de sels solubles des métaux Cu Co Zn ainsi qu'éventuellement Zr et/ou M et/ou N, portée à une température comprise entre 0 et 30 ° C et contenant au moins un atome gramme de l'ensemble des métaux (Cu + Co + Zn + Zr + M + N) par litre avec une solution de carbonate et/ou d'hydrogènocarbonate (bicarbonate) et/ou d'hydroxyde de préférence de sodium et/ou de potassium et/ou d'ammonium à une température comprise entre 0 et 30 ° C et contenant au moins deux atomes gramme de cations alcalins et/ou d'ammonium $NH_4^+$ par litre, la réaction de coprécipitation étant menée entre 0 et 30 ° C, le pH mesuré dans le volume réactionnel étant fixé à 7 ± 1 unité pH, et le temps de résidence du mélange (coprécipité + eaux mères) dans le volume réactionnel n'excédant pas cinq minutes.

On obtient de la sorte un hydroxycarbonate mixte hydraté homogène, amorphe en diffraction X, donnant par diffraction X et enregistrement goniométrique un diagramme "plat". Ce produit est ensuite lavé de façon à réduire sa teneur en métaux alcalins ou ammonium (exprimée en poids par rapport au poids total de métaux) à 0,05 - 5%, et de préférence de 0,09 à 3,5% poids de façon à obtenir après activation thermique finale une concentration en métaux A au plus égale à celle souhaitée dans le catalyseur prêt à l'emploi.

A cet effet, les techniques de désalcalinisations connues de l'homme du métier et, notamment, celles décrites par la demanderesse dans la demande de brevet français FR-A-2 558 738 (correspondant à la demande des Etats Unis S.N. 695,021 déposée le 21 janvier 1985) peuvent avantageusement être utilisées.

Un autre mode préféré de réalisation de l'invention consiste à faire réagir à une température d'au moins 30 et de préférence d'au moins 50 ° C, et d'une manière la plus préférée d'au moins 70 ° C une solution de sels solubles des métaux Cu Co Zn éventuellement Zr et/ou M et/ou N, de concentration globale au plus égale à 1 at.g de métaux par litre, par exemple entre 0,1 et 1 at.g de métaux par litre, avec une solution de carbonate et/ou d'hydrogénocarbonate et/ou d'hydroxide, de préférence de sodium et/ou de potassium, et/ou d'ammonium de concentration globale au plus égale à 2 at.g (par exemple comprise entre 0,1 et 1,5 at.g) de métaux alcalins et/ou $NH_4^+$par litre, la réaction de coprécipitation étant menée à un pH de 7 ± 1 unité pH, et le temps de résidence dans le milieu réactionnel étant d'au moins 2 minutes; on obtient de la sorte un hydroxycarbonate mixte hydraté, homogène, cristallisé au moins en partie.

Le composé cristallisé peut éventuellement ensuite être mûri par exemple entre environ 15 ° C et environ 100 ° C à pression atmosphérique ou encore entre environ 100 et environ 250 ° C dans un autoclave opérant sous pression, pendant 15 minutes à 5 heures, en présence de ses eaux mères ou encore de ses eaux de lavage. Au cours de cette opération de mûrissement, on note en général une augmentation du pH, généralement au plus égale à 1,5 unité pH, par rapport au pH de précipitation. On observe que, d'une façon inattendue ce traitement de mûrissement améliore la cristallinité et/ou augmente la taille des cristallites du précurseur hydraté cristallisé.

L'opération de mûrissement, si la précipitation est faite en "batch" peut être menée dans le même réacteur, après arrêt de l'injection des réactifs. On peut également, dans le cas de la précipitation continue, recueillir le précipité obtenu en conditions stationnaires (température, concentrations, pH, vitesse d'introduction des réactifs) et le mûrir, après lavage éventuel, dans un autre réacteur ou encore dans un autoclave.

De préférence, pour la préparation de l'hydroxycarbonate mixte cristallisé, la température réactionnelle sera d'au moins 70 ° C et le pH de 7 ± 0,3 unités pH, la concentration de la solution de sels des métaux Cu, Co, Zn, Zr, M et N, sera comprise entre 0,1 et 0,6 at.g de métaux par litre et celle des composés des métaux alcalins et/ou de l'ion ammonium entre 0,2 et 1,2 at.g de métaux alcalins et/ou ammonium par litre, et le temps de réaction sera d'au moins 5 minutes.

Après précipitation et mûrissement éventuel dans les eaux mères, le précipité cristallisé est lavé de façon à réduire sa teneur en métal A (exprimée en poids de métal A par rapport au poids total de métaux) à 0,01 - 0,4% poids et d'une manière préférée à 0,05 - 0,2% poids puis mûri éventuellement dans les eaux de lavage. On peut également laver de façon à réduire la teneur en métal A par exemple à 0,09-3,5%, par exemple dans le cas ou l'on ne désire pas effectuer d'alcalinisation ultérieure.

Un autre mode préféré de préparation des catalyseurs employés dans le procédé selon l'invention consiste à réaliser, en particulier dans le cas où le catalyseur contient du zirconium et éventuellement au moins un métal M, une précipitation en deux étapes distinctes, puis un mélange des deux coprécipités de manière à former une dispersion sensiblement homogène. Cette préparation comprend les étapes suivantes : a) on prépare par l'une au moins des procédures de coprécipitation décrites ci-avant, un précurseur hydraté contenant le cuivre, le cobalt, au moins une partie du zinc (par rapport à la totalité du zinc que l'on

souhaite introduire dans le catalyseur que l'on veut préparer) et éventuellement au moins un métal N; b) le précurseur hydraté, cristallisé au moins en partie obtenu à l'étape (a) est alors lavé à l'eau de manière à obtenir un produit contenant moins de 0,2% en poids environ de métaux alcalins par rapport au poids total des métaux présents; c) on prépare par coprécipitation à chaud c'est-à-dire à une température supérieure à 30 °C environ et de préférence supérieure à 50 °C et d'une manière la plus préférée supérieure à 70 °C, de la manière décrite ci-avant, un précurseur hydraté contenant le zirconium, éventuellement le complément en zinc nécessaire et éventuellement au moins un métal M, ces divers métaux étant engagés à partir d'au moins un des composés solubles précités, l'agent coprécipitant étant au moins un composé du cation ammonium (hydroxydes, carbonate, bicarbonate). Le pH de coprécipitation est compris entre 6 et 8 unités pH environ; après coprécipitation, le composé hydraté contenant les métaux mentionnés ci-dessus, peut éventuellement être mûri dans les conditions opératoires décrites ci-avant. Après précipitation et mûrissement éventuel dans les eaux mères, le précipité contenant le zirconium et éventuellement une partie du zinc et éventuellement au moins un métal M est lavé (étape d) à l'eau de façon à réduire sa teneur en azote ($NH_4^{+5}$ et éventuellement $NO_3^-$) à une valeur, rapporté au poids total des métaux inférieurs à 3% et préférentiellement inférieurs à 1% poids.

Les deux coprécipités lavés obtenus comme il vient d'être dit sont ensuite mélangés (étape e) dans un appareillage permettant d'obtenir une dispersion aussi homogène que possible des deux produits l'un dans l'autre. La dispersion peut être mesurée à la microsonde de Castaing ou encore par microanalyse X au microscope à balayage (STEM). Les meilleurs résultats sont obtenus pour une dispersion sensiblement homogène à l'échelle 0,01-0,1 (micron).

La dispersion sensiblement homogène des deux coprécipités est obtenue, par utilisation de leurs propriétés thixotropes, en soumettant le mélange à des forces de cisaillement suffisamment importantes, appliquées au produit par l'intermédiaire de pales tournantes, de disques, de cylindres ou encore par passage au travers d'orifices, par exemple dans le mélangeurs Werner, Cowles, Waring, Hobart, Hockmeyer, Rousselle, et dans certains mélangeurs à galets. Après arrêt du malaxage cisaillant, aucune décantation et/ou ségrégation ne doit être observée.

Le séchage du produit mixte homogénéisé peut être réalisé par tout procédé connu; on peut l'effectuer par exemple par atomisation (spray-drying). On obtient un produit sensiblement homogène, en poudre calibrée contenant d'environ 60 à environ 80% en poids d'équivalent d'oxydes. On peut également sécher la produit en étuve, par exemple entre environ 50 et environ 150 °C, sous balayage d'air, de façon à ramener si nécessaire la teneur en oxydes potentiels à environ 60 à 80% en poids. Il est recommandé d'éviter la stagnation du précipité en présence de pressions partielles de vapeur d'eau proches de la pression de vapeur saturante à la température de séchage considérée; de tels traitements peuvent avoir pour conséquence une déshydratation partielle du précipité avec cristallisation d'oxyde cuivrique en gros cristallites. Un séchage combiné par atomisation suivi d'un séchage en étuve est également possible.

Des exemples détaillés de cette technique de préparation sont donnés dans le brevet US-A-4 552 861 de la demanderesse.

Après précipitation et lavage, selon l'une au moins des procédures de préparation qui viennent d'être décrites, on obtient un précurseur hydraté (cristallisé ou amorphe) homogène, contenant environ de 10 à environ 30% poids d'oxydes s'il est amorphe et de environ 15 à environ 60% poids d'oxydes s'il est cristallisé.

Dans ce précurseur amorphe ou cristallisé, la répartition des métaux est homogène et les rapports atomiques Zn/Co et éventuellement Zr/Co varient de moins de 15% (relatifs) et préférentiellement de moins de 10% à l'échelle 5 nm .

Dans les précurseurs mixtes obtenus par une précipitation en deux étapes distinctes et qui sont formés par une juxtaposition de particules homogènes de tailles comprises entre environ 3 et environ 100 nm, et de compositions différentes, les mesures des rapports zinc/cobalt et éventuellement zirconium/cobalt seront faites à forte résolution sur les particules individualisées contenant le cobalt.

Les particules ne contenant pas le cobalt sont, de même, homogènes entre elles.

Un premier mode d'ajout éventuel du ou des métaux A (au moins un alcalin et/ou alcalino-terreux) consiste à mettre en contact le précipité hydraté avec une solution contenant le ou les métaux A puis à agiter vigoureusement de façon qu'après mise en suspension du précipité dans la solution alcaline puis mûrissement et/ou filtration éventuels, ce dernier contienne au moins un des métaux A précités en proportion convenable.

Le séchage de la suspension de précipité et des métaux A en solution, après mûrissement éventuel, peut par exemple être effectué par atomisation (spray-drying) et/ou en étuve. On obtient alors un précipité alcalinisé séché en poudre calibrée constituée de sphéroïdes creux (cénosphères) de diamètre 3-700 micromètres et de composition homogène, contenant de environ 60 à environ 85% poids d'oxydes, soit

amorphe s'il est issu d'un précurseur hydraté amorphe, soit cristallisé s'il est issu d'un produit cristallisé.

On peut également séparer le précipité du milieu alcalinisant par filtration, puis ensuite le mûrir éventuellement, puis le sécher, par exemple par atomisation et/ou par séchage en étuve afin de ramener sa teneur en oxydes à environ 65-85% poids.

Un autre mode d'alcalinisation consiste à ajouter au moins un métal A sous forme d'une solution aqueuse et/ou organique qui peut être mélangée avec le précipité séché (précipité amorphe, séché par atomisation, ou précipité cristallisé). On obtient une pâte homogène qui est ensuite séchée par toute technique appropriée; par exemple, comme il vient d'être dit ci-avant.

Ce précipité est ensuite activé thermiquement comme il va l'être dit, mais ce traitement d'activation peut également être conduit sur un précipité soigneusement désalcalinisé par lavage (amorphe ou cristallisé) ne contenant pas encore au moins un métal A et présentant les caractères d'homogénéité précités.

L'activation thermique consiste à traiter le précipité séché, alcalinisé ou non encore alcalinisé, à une température d'environ 250 à environ 600°C et de préférence entre environ 300 et environ 500°C pendant une durée suffisante, par exemple pendant au moins 0,5 heure pour obtenir un catalyseur activé homogène ne renfermant pas plus de 12% poids de matières volatiles (le taux de matières volatiles est mesuré par exemple par activation en présence d'air d'un poids donné de produit, placé dans une nacelle et calciné à 600°C pendant 4 heures).

L'activation thermique peut être menée selon les cas, soit en présence d'un gaz inerte contenant de 0 à 50% d'oxygène, on obtient alors un oxyde mixte homogène, soit en présence d'un milieu réducteur (mélanges gaz inerte - gaz réducteur renfermant 0,1 à 100% de gaz réducteur); les gaz réducteurs, employés seuls ou en mélange sont par exemple l'hydrogène ou l'ammoniac.

L'activation thermique en milieu globalement réducteur peut être menée soit sur le précurseur séché, soit sur l'oxyde mixte préalablement activé en milieu globalement oxydant.

Après activation thermique en milieu globalement réducteur, l'oxyde mixte peut être partiellement réduit (par le gaz contenant de l'hydrogène) ou encore partiellement réduit et nitruré (par le gaz contenant de l'ammoniac).

Le catalyseur activé thermiquement puis éventuellement broyé peut ensuite être éventuellement mis en contact avec une solution aqueuse ou organique d'au moins un métal N de façon à disperser d'une manière essentiellement uniforme le dit métal, et à obtenir après sèchage et activation thermique comme ci-avant un catalyseur où le dit métal est bien dispersé (la dispersion peut par exemple se mesurer par chimisorption de gaz réactifs CO, $H_2$, sur le dit métal). A l'exception des halogénures et des sulfates, tous les sels solubles, par exemple nitrates, acétylacétonates, ainsi que les complexes par exemple nitrosamminés, amminés, carbonylés sont utilisables.

Le précipité hydraté (amorphe ou cristallisé), homogène, soigneusement désalcalinisé, séché de façon à réduire sa teneur en matières volatiles à moins de 35% poids puis activé thermiquement, éventuellement imprégné par au moins un métal N, peut être enfin alcalinisé comme suit : le produit homogène résultant de l'activation thermique est broyé de manière à obtenir une poudre de granulométrie inférieure ou égale à 0,2 mm, puis l'agent alcalinisant (au moins un métal A) est ajouté par exemple par malaxage du dit produit avec une solution aqueuse et/ou organique contenant au moins un composé d'au moins un métal A dans les proportions précitées.

Après ajout de l'agent alcalinisant, la pâte peut éventuellement être mise en forme par extrusion (on peut ainsi obtenir, après sèchage et activation, des extrudés de bonnes propriétés mécaniques), puis elle est séchée par toute technique connue. Avant sèchage il peut être avantageux d'effectuer un mûrissement à l'air ambiant pendant un temps suffisant pour ramener la teneur en eau du produit à moins de 25% et, de préférence, à moins de 20% poids.

Un autre mode d'addition de l'agent alcalinisant et de mise en forme simultanée consiste à placer la poudre précitée dans une turbine à dragéifier puis à pulvériser sur la poudre, animée d'un mouvement de rotation, la solution aqueuse ou organique contenant l'agent alcalinisant. On peut obtenir de la sorte des billes calibrées (par exemple entre 2,4 et 5 mm) qui, après mûrissement éventuel, séchage et activation thermique, conduisent à un catalyseur activé, homogène, de bonnes propriétés mécaniques.

Un autre procédé permettant de combiner l'opération d'alcalinisation et l'opération de mise en forme consiste à faire une suspension contenant le catalyseur activé, à une teneur d'environ 30-60% poids et au moins un métal A, à procéder ensuite, éventuellement à un mûrissement, puis à faire une calcination flash dans un atomiseur opérant en présence d'un gaz de combustion contenant moins de 1 mg de soufre par N.M3 et présentant une température d'entrée de 500°C au moins. On obtient ainsi des microsphères de 2 à 700 micromètres qui peuvent éventuellement être utilisées dans un procédé en phase liquide avec circulation du catalyseur.

Au cours d'une au moins des opérations unitaires où le précurseur hydraté, ou le précurseur hydraté séché, ou le catalyseur activé est mis au contact avec au moins une phase liquide, il peut être avantageux de procéder à un traitement aux ultrasons du milieux mixte (solide + liquide) afin de parfaire leur interaction, de procéder au moins en partie à une fragmentation du solide et/ou de contrôler au moins en partie les phénomènes de modification de l'état cristallin.

Le traitement aux ultrasons peut évidemment être couplé à l'opération de coprécipitation; dans ce dernier cas le réacteur contient au moins un dispositif générateur d'ultrasons qui est mis en fonctionnement pendant l'opération unitaire de coprécipitation et/ou pendant le mûrissement éventuel.

Ce traitement peut également être avantageusement appliqué aux suspensions comprenant au moins un coprécipité, précurseur hydraté, en présence éventuellement d'au moins un métal A alcalin et/ou alcalino-terreux; il peut permettre d'améliorer la dispersion, et/ou l'homogénéité et/ou il peut faciliter le sèchage ultérieur lorsque ce dernier est pratiqué par atomisation.

Ou pourra par exemple au laboratoire employer les générateurs ULTRASONICS inc. (USA) modèles W 220 F, W 225 R, la fréquence émise correspond à 20 KHz (20 000 cycles par seconde) sous une puissance de 10 à 200 watts. Cette puissance dissipée est suffisante pour traiter les volumes de produits au plus égaux à 15 litres. Des appareils plus puissants pourront avantageusement être utilisés pour le traitement de volumes supérieurs.

Le catalyseur alcalinisé, préparé comme il vient d'être dit (pâte homogène, extrudés homogènes, billes homogènes, microsphères homogènes) éventuellement mûri, éventuellement séché si nécessaire de façon à ramener sa teneur en matières volatiles à moins de 35% poids et préférentiellement à moins de 25% poids, est enfin activé thermiquement dans les conditions et en présence des réactifs gazeux précités.

Cependant cette deuxième activation thermique sera menée de préférence entre environ 300 et environ 450 °C pendant un temps suffisant pour ramener la teneur du produit en matières volatiles à moins de 12% poids.

Si le catalyseur alcalinisé puis activé thermiquement dans les conditions précitées n'a pas encore été mis en forme, il le sera comme suit :
le produit homogène, activé thermiquement, est broyé, par exemple en dessous de 0,5 mm, mélangé à raison de 0,5-5% de son poids avec au moins un composé adjuvant de pastillage choisi dans le groupe formé par le graphite, l'acide stéarique, les stéarates, et éventuellement un adjuvant de porosité choisi parmi la cellulose et les poudres d'origine végétale en contenant, le nitrate et les carbonates d'ammonium, les fibres textiles combustibles, le naphtalène, puis enfin pastillé en cylindres pleins de diamètres 3-6 mm ou en cylindres toriques de diamètres extérieur 3-6 mm et intérieur 1 à 4 mm et de hauteur 2 à 6 mm.

Le catalyseur mis en forme par pastillage subira éventuellement une ultime activation thermique dans les conditions opératoires précitées.

Le catalyseur activé thermiquement et prêt à l'emploi est constitué par une association très homogène d'oxydes (éventuellement certains d'entre eux peuvent être réduits, au moins en partie, si au moins une activation thermique a été menée en milieu globalement réducteur). Dans cette association très homogène d'oxydes, les métaux et notamment le cobalt, le zinc et le zirconium si le catalyseur en contient, sont répartis à l'échelle de 5 nm de manière très homogène, et les variations relatives des rapports atomiques Zn/Co et éventuellement Zr/Co sont inférieures à 15% et préférentiellement à moins de 10%. La surface spécifique des dits catalyseurs varie entre environ 20 et environ 300 $m^2g^{-1}$ .L'activation thermique, lorsqu'elle est menée en présence d'au moins un métal A alcalin et/ou alcalinoterreux, et à une température d'au moins 350 °C, peut provoquer la recristallisation de particules individualisées d'oxyde cuivrique CuO (ténorite) visibles en diffraction X, sans que ceci modifie la dispersion du cobalt par rapport aux autres métaux Zn, Zr, M, N, et A précités.

Les conditions de mise en oeuvre des dits catalyseurs pour la fabrication des alcools sont habituellement les suivantes :
le catalyseur, chargé au réacteur, est tout d'abord réduit par un mélange de gaz inerte (azote par exemple) et d'au moins un composé réducteur choisi dans le groupe formé par l'hydrogène, le monoxyde de carbone, les alcools et les aldéhydes en $C_1$ et $C_2$, le rapport molaire composé réducteur/composé réducteur + gaz inerte étant de 0,001 : 1 à 1 : 1.

La température de réduction varie généralement de 100 à 350 °C mais de préférence entre 130 et 320 °C. La pression totale est habituellement comprise entre 0,1 et 5 MPa et de préférence entre 0,2 et 2 MPa; la vitesse volumétrique horaire est habituellement de $0.5.10^2$ à $1.10^4$ heure $^{-1}$ et de préférence de $1.10^2$ à $5.10^3$ heure $^{-1}$ (T.P.N.).

D'une façon la plus préférée, la réduction sera menée par exemple entre environ 150 et environ 300 °C, en présence du mélange réducteur précité et avec un rapport molaire gaz réducteur/gaz réducteur + gaz inerte compris entre 0,001 : 1 et 0,1 : 1 et préférentiellement entre 0,005 : 1 et 0,05 : 1, la température de

réduction étant augmentée de façon progressive (par exemple de 20 en 20 ° C) et chaque augmentation de température étant suivie d'un palier isotherme, à température fixe, pendant un temps suffisant pour que les concentrations en gaz réducteur soient les mêmes à l'entrée et à la sortie du réacteur (ce qui prouve que la réduction à la température du palier considéré est terminée).

La réduction du catalyseur peut également être menée en phase liquide si, par la suite, la réaction de synthèse d'alcools se déroule en phase liquide. Les conditions opératoires restent inchangées, mais la vitesse de montée en température et/ou la concentration en gaz réducteur dans le mélange gazeux peuvent être plus importants, tout en restant à l'intérieur des fourchettes précitées.

Le catalyseur réduit comme il vient d'être dit, peut enfin être mis en contact avec le gaz de synthèse ($CO + H_2 + CO_2$). Il est connu de l'homme du métier que cette mise en contact doit être progressive. La température opératoire initiale est en général supérieure à environ 210 ° C et préférentiellement à environ 230 ° C.

Les conditions de pression opératoire et de vitesse volumétrique horaire sont celles décrites ci-après.

La réaction proprement dite de synthèse des alcools est réalisée dans les conditions opératoires suivantes :

La pression totale est habituellement comprise entre 2 et 25 MPa et de préférence entre 5 et 15 MPa.

La pression partielle en ($H_2 + CO + CO_2$), sera généralement inférieure à la pression totale; cette différence provient de l'accumulation, par le recyclage des gaz non transformés, des gaz inertes ($CH_4$, $N_2$, gaz rares) apportés par le gaz d'appoint ainsi que de l'accumulation des sous produits hydrocarbonés de la réaction (hydrocarbures en $C_1$, $C_2$, $C_3$ essentiellement).

La pression partielle en ($H_2 + CO + CO_2$) sera habituellement comprise entre 2 et 15 MPa et, de préférence entre 5 et 12 MPa.

Comme il va être dit ci-après, le rapport molaire moyen $H_2/CO$ dans la zone de réaction; défini comme la moyenne des rapports entrée et sortie réacteur, est de 0,1 : 1 à 4 : 1, mais de préférence de 0,2 : 1 à 3,5 : 1 ; la température moyenne dans le réacteur est de 250 à 350 ° C et, de préférence, de 260 à 320 ° C.

La vitesse volumique horaire (exprimée en volume TPN de mélange gazeux par volume de catalyseur et par heure) est usuellement comprise entre 1000 et 40.000 $h^{-1}$ et de préférence entre 2000 et 20.000 $h^{-1}$.

Le catalyseur peut être utilisé en poudre fine calibrée (10 à 700 micromètres) ou en particules de diamètre équivalent 2 à 10 mm, en présence d'une phase gazeuse, ou d'une phase liquide (dans les conditions opératoires) et d'une phase gazeuse. La phase liquide peut être constituée par un ou plusieurs hydrocarbures ayant au moins 5 et de préférence au moins 10 atomes de carbone.

Dans ce mode de mise en oeuvre, il est préférable que les vitesses superficielles du gaz et du liquide, dans les conditions de température et de pression du procédé, soient d'au moins 1,5 cm/sec. et de préférence d'au moins 3 cm/sec. Par vitesse superficielle on entend le rapport du débit volumique à la section du réacteur considéré vide de catalyseur.

La synthèse des alcools étant une réaction très fortement exothermique, il est important que les conditions opératoires (température, pression, composition du mélange réactionnel, vitesse volumétrique horaire VVH) soient ajustées de façon à limiter la transformation chimique du mélange réactionnel ($CO + H_2$) en présence des catalyseurs précités, et les effets thermiques qui en découlent.

Pour cette raison, il peut être avantageux de limiter, la conversion (par passe) du monoxyde de carbone (CO) à environ 5-25% et, après condensation des liquides produits par la réaction, de procéder au moins en partie à un recyclage des gaz non transformés de façon à transformer globalement 85 à 95% du CO contenu dans le gaz d'appoint.

Cette mise en oeuvre est notamment connue et appliquée par l'homme du métier, pour le procédé industrialisé de synthèse du méthanol à partir de $CO + CO_2 + H_2$.

Les réacteurs utilisés pour la synthèse des alcools en présence des catalyseurs de l'invention peuvent être soit isothermes (il s'agit alors en général d'un réacteur multitubulaire cù le catalyseur est placé à l'intérieur des tubes, et où la chaleur de réaction est transférée à un fluide thermique approprié) soit adiabatique.

Tous les réacteurs adiabatiques permettant à la fois de disposer la quantité maximum de catalyseur dans un volume total minimum; de limiter les élèvations de températures dans les grains et/ou dans le lit de catalyseur, et de limiter les pertes de charge dans le réacteur sont utilisables. Ces réacteurs peuvent contenir un ou plusieurs lits de catalyseur.

L'écoulement du gaz de synthèse dans le réacteur peut être soit axial, soit radial, soit mixte. Une partie du gaz de synthèse et/ou des produits gazeux de réaction, après refroidissement convenable,peut être injecté entre les lits de catalyseur (refroidissement par trempe ou quench). La chaleur de réaction peut également être éliminée, de façon classique par des échangeurs intermédiaires soit externes, soit internes au réacteur.

La réaction de synthèse peut être menée avec un ou plusieurs réacteurs, disposés en série et/ou en parallèle; dans ce dernier cas les produits de réaction peuvent avantageusement être condensés intermédiairement, avant admission du gaz non transformé dans le réacteur suivant.

Dans la réaction de synthèse produisant du méthanol, des alcools supérieurs, de l'eau et, en moindre proportion,des hydrocarbures, une partie du CO peut se transformer en $CO_2$ selon la réaction (équilibrée) de conversion du CO.

$$CO + H_2O \rightleftharpoons CO_2 + H_2$$

Les catalyseurs, objets de la présente invention, présentent également une très bonne activité pour la réaction de conversion du monoxyde de carbone selon la réaction ci-avant.

Afin de limiter la quantité d'eau formée, en parallèle aux alcools produits, il peut enfin être avantageux d'enlever une partie du $CO_2$ formé lors de la réaction (par exemple par décarbonatation par un milieu solvant de $CO_2$ approprié) après condensation des liquides et avant de procéder au recyclage, au moins en partie, du gaz précité.

Comme la réaction de conversion à l'eau du CO est une réaction équilibrée :

$$CO + H_2O \rightleftharpoons CO_2 + H_2$$

$$K_{(T)} = \frac{P_{CO_2} \cdot P_{H_2}}{P_{CO} \cdot P_{H_2O}}$$

On voit qu'à une température T, la pression partielle d'eau $P_{H2O}$ à la sortie du réacteur sera fonction de la pression partielle de $CO_2$, du rapport $H_2/CO$, et sera d'autant plus faible que ces deux valeurs seront elles-mêmes plus faibles;

$$P_{.H_2O} = \left(\frac{H2}{CO}\right) \cdot \frac{P_{CO_2}}{K_T}$$

comme par ailleurs, la constante $K_T$ augmente fortement dès que la température diminue, il sera avantageux de disposer de catalyseurs particulièrement actifs pour opérer aux températures les plus basses.

Bien que les catalyseurs selon l'invention puissent être utilisés en présence de gaz de synthèse ($H_2$ + CO + $CO_2$) contenant jusqu'à 15%et préférentiellement jusqu'à 10% en volume de $CO_2$, il peut être avantageux de limiter la concentration moyenne en $CO_2$ à 0-5% et préférentiellement à 0,1-3% en volume dans le réacteur, de façon à transformer par conversion un maximum de l'eau formée en parallèle avec les alcools et/ou les hydrocarbures et à simplifier la procédure de déshydratation des dits alcools. Les alcools bruts alors produits contiennent en général de 0,1 à 10% poids d'eau et plus particulièrement de 0,5 à 5% poids .

Afin de réaliser le même objectif, il peut être également avantageux d'ajuster le rapport molaire $H_2/CO$ dans le réacteur entre 0,2 et 3,5 et plus particulièrement entre 0,5 et 2,8.

Les exemples qui suivent décrivent différents aspects de l'invention, sans toutefois en limiter la portée.

On décrit tout d'abord la préparation des catalyseurs A à J dont les caractéristiques figurent dans le tableau 1. La densité de remplissage est déterminée pour un réacteur de diamètre intérieur 2,5 centimètres.

Catalyseur A.

On dissout dans l'eau 193,3 g de nitrate cuivrique trihydraté (0,8 at.g. Cu) 174,62 g de nitrate de cobalt hexahydraté (0,6 at.g. Co), 267,27 g de nitrate de zirconyle dihydraté (1,0 at.g.Zr) en présence de 0,3 litre

d'acide nitrique pur (d= 1,38) et 148,74 g de nitrate de zinc hexahydraté (0,5 at.g.Zn). La solution (solution A, 0,48 at.g/l) est étendue à 6 litres.

On dissout séparément 640 g de carbonate discdique anhydre dans 10 litres d'eau (solution B, 1,2 at.g. Na/litre).

Les deux solutions portées à 75° C sont ajoutées simultanément dans un réacteur de 2,0 litres, contenant 1,5 litres d'eau portée à 75° C, ce réacteur est muni d'une surverse, les débits des deux solutions sont régulés par le pH, mesuré dans la zone de turbulence de la turbine d'agitation (turbine STARO). La durée de précipitation est de 1 heure 30 minutes et le temps de séjour moyen de 12,4 minutes; le pH varie entre 6,95 et 7,04 unité pH. Le précipité mûri 1 heure à 40° C en présence de ses eaux mères est ensuite lavé par 36 litres d'eau bipermutée (3 lavages successifs par 12 litres d'eau). Le précipité lavé contient 28% en poids d'oxydes et 0,04% en poids de sodium par rapport aux métaux. 750 g de précipité lavé (210 g d'oxydes équivalents) sont placés dans un malaxeur HOBBART, mélangés avec 0,05 l d'eau contenant 1,09 g de carbonate dipotassique (0,016 at.g.K) et 2,44 g de carbonate disodique (0,046 at.g.Na). Le mélange est malaxé deux heures, puis mûri 10 heures à 25° C avant séchage par atomisation (Température de sortie : 150° C, temps moyen de séjour t=0,9 seconde). Le produit obtenu qui contient 77% en poids d'oxydes est activé thermiquement sous air à 350° C pendant 4 heures, mélangé avec 2% en poids de graphite naturel puis pastillé en cylindres de diamètre et de hauteur 4 mm. Après activation thermique (300° C 2 heures sous azote), on obtient 190 g de pastilles dont la densité de remplissage est de 1,12 kg/l.

Catalyseur A1

Ce catalyseur est préparé dans les mêmes conditions opératoires que celles décrites pour la préparation du catalyseur A;on prépare ainsi 150g. d'oxydes avant alcalinisation. L'alcalinisation, effectuée à l'aide d'une solution aqueuse de carbonate disodique, est suivie d'un mûrissement hydrothermique en récipient fermé (T=60° C,t=4h.), puis le produit est séché, activé, pastillé et réactivé comme décrit ci-avant pour la préparation du catalyseur A. On obtient 80g. de pastilles (3,5x4mn) de densité de remplissage égale à 1,15 kg/l.

Catalyseur B

On prépare 12 litres d'une solution contenant 410,75 g de nitrate cuivrique trihydraté (1,7 at.g.Cu) 232,85 g de nitrate de cobalt hexahydraté (0,8 at.g.Co) 297,50 g de nitrate de zinc hexahydraté (1 at.g.Zn) 79,45 g d'hydroxynitrate cérique trihydraté (0,2 at.g.Ce). Cette solution A contient 0,308 at.g. de métaux par litre, elle est préchauffée à 75° C.

On dissout séparément 549 g de carbonate disodique dans 17 litres d'eau bipermutée préchauffée à 70° C (solution B, 0,61 at.g. Na/litre).

Dans un réacteur de 50 litres contenant 5 litres d'eau bipermutée chaude (70° C), muni d'un chauffage externe, d'une agitation (hélice, 700 t/mn) et en présence du générateur d'ultrasons W 220 F (puissance absorbée 150 watts) et d'une régulation du pH avec asservissement des débits au pH, les deux solutions sont ajoutées de façon simultanée; l'opération dure 3 heures. En fin d'opération, le précipité est refroidi, puis mûri 5 heures à 40° C et finalement lavé sur un filtre presse de laboratoire.

La teneur finale en alcalin du précipité lavé est de 0,025% poids par rapport aux métaux, le produit lavé est cristallisé et homogène; il contient 33% poids d'oxydes.

Après malaxage comme ci-avant pour la préparation du catalyseur A, mais avec 3,045 g de $K_2CO_3$ - (0.045 at.g.K) et 0,705 g de $Rb_2\ CO_3$ (0,006 at.g. Rb) dans 0,1 l d'eau, pendant 30 mn, et mûrissement 60 heures à 15-22° C, le précipité alcalinisé est séché par atomisation (temps moyen de séjour : 1,5 seconde; température de sortie : 130° C) activé thermiquement (T= 320° C t= 10 heures) puis pastillé après ajout de 1,5 % de stéarate de magnésium. Après l'ultime activation thermique (t= 350° C t= 1 heure), on obtient 302 g de pastilles 4 x 4 mm de densité de remplissage 1,18 $kg.l^{-1}$

Catalyseur C

On prépare tout d'abord l'oxyde mixte de formule $Nd_{0,2}Zr_{0,6}O_{1,8}$.

0,2 mole de nitrate de zirconyle dihydraté (160,36 g) sont dissouts dans 2 litres d'eau, en présence de 0,15 l d'acide nitrique pur (d= 1,38) et chauffés à 85° C.

0,36 kg d'une solution de carbonate diammonique $(NH_4)_2CO_3$ à 30% en poids de sel cristallisé sont dilués dans 2 litres d'eau froide; La solution est ensuite chauffée à 65° C. On ajoute simultanément les deux solutions dans un réacteur opérant en continu (suivant la méthode décrite pour le catalyseur A). Le temps

de séjour moyen est de 20 mn, le pH varie entre 6,5 et 6,8 unités pH, le produit est directement lavé 3 fois par 18 litres d'eau chaude bipermutée.

On obtient 530 g de précipité humide (107 g d'oxydes) contenant moins de 0,3% en poids d'azote.

En parallèle, on prépare 223,44 g du catalyseur de formule $Cu_1$ $Co_{0.4}$ $Zn_{1,4}$ $O_{2,8}$ qui contient 35,57% Cu; 13,19% Co; 51,24% Zn par rapport aux métaux, selon la procédure décrite pour le catalyseur A et ce, jusqu'au mûrissement consécutif au lavage. On obtient 860 g de précipité lavé (0,04% Na par rapport aux métaux) contenant 223,44 g d'oxydes.

Les deux précipités (Nd Zr) et (Cu Co Zn) sont placés dans un malaxeur WARIG BLENDER et agités 3 heures à température ambiante. La suspension thixotropique obtenue est alors traitée par 0,1 litre d'une solution contenant 8,96 g de $Na_2CO_3$ (0,169 at.g.Na) et 0,740 g de $Cs_2$ $CO_3$ (0,0045 at.g. de Cs), mûri sous agitation lente pendant 6 autres heures à l'ambiante, puis séchée par atomisation (t= 120-140 °C - temps = 2 secondes).

On obtient 398 g de produit séché, et homogène contenant 75% en poids d'oxydes. Ce produit, calciné 5 heures à 400 °C est mélangé avec 2,5% de graphite naturel, puis pastillé en pastilles toriques de diamètre extérieur et de hauteur 5 mm et de diamètre intérieur 2,5 mm. La densité de remplissage du produit est de 1,05 kg/l.

L'examen au microscope électronique à balayage par transmission (MEBT ou mode STEM dans la terminologie anglo-saxonne) du catalyseur montre qu'il est constitué de deux phases différentes l'une contenant du cuivre, du cobalt et du zinc et où le rapport Zn/Co dans les particules contenant du cobalt varie entre 3,4 et 3,8, et l'autre contenant du zirconium et du neodyme également très homogène entre eux. Le sodium et le césium sont également répartis de façon homogène.

Catalyseur D1

314,08 g de nitrate cuivrique trihydraté (1,3 Cu) 145,52 g de nitrate de cobalt hexahydraté (0,5 Co) et 357 g de nitrate de zinc hexahydraté (1,2 Zn) sont dissouts dans 2 litres d'eau bipermutée; on ajoute ensuite 420 g d'acide citrique monohydraté (2 moles) et finalement 1,18 g de citrate de sodium trisodique dihydraté (0,012 at.g.Na). Après évaporation sous vide (70 °C, 0,01 MPa) pendant 5 heures, la substance vitreuse obtenue est broyée puis introduite progressivement dans un tube vertical chauffé à 500 °C parcouru par un courant d'air sec. Le temps de séjour moyen et de l'ordre d'une minute. La poudre divisée est enfin calcinée sous balayage d'air en four statique (400 °C -3 heures) compactée, pastillée et recalcinée (300 °C - 1 heure); on obtient 165 g de pastilles de diamètre et de hauteur 3 mm. Leur densité de remplissage est de 1,35 $kg.l^{-1}$. La surface spécifique mesurée par la méthode BET est de 27$m^2$. $g^{-1}$.

Catalyseur D2

Les mêmes quantités des mêmes sels que ceux employées pour la préparation du catalyseur D1 (à l'exception de l'acide citrique et du citrate de sodium) sont dissoutes dans de l'eau bipermutée froide; on prépare 4,5 litres de solution (0,67 at.g/l). On prépare séparément 6 litres d'une solution aqueuse (20 °C) contenant 238,5 g de carbonate disodique.

Les deux solutions sont mises en contact dans l'appareillage décrit pour la préparation du catalyseur A. Le temps moyen de résidence dans le coprécipitateur est de 15 minutes; le pH varie entre 7,02 et 7,07 unités pH. Après filtration subséquente, le précipité est immédiatement mis en suspension dans 12 litres d'eau bipermutée froide (agitation 30 mn - turbine RAYNORI) cette opération est répétée 8 fois.

On obtient (compte tenu des pertes) 140 g d'oxydes sous forme d'un coprécipité hydraté, amorphe en diffraction X et très homogène, sa teneur en sodium (sodium résiduel, ex précipitation) est de 0,15% poids par rapport aux métaux.

Le précipité est malaxé 30 minutes (malaxeur BECKEN à lames sigmoïdes) puis séché à l'étuve à 60 °C pendant 5 heures, puis à 90 °C pendant 3 heures et finalement à 120 °C pendant 2 heures. Il est ensuite activé thermiquement sous air (420 ° - 10 heures), pastillé (ajout 2% de graphite), réactivé à 320 °C pendant 2 heures. On obtient 120 g de pastilles de diamètre et hauteur 3,0 mm, leur densité de remplissage et de 1,3 $kg.l^{-1}$ et leur surface spécifique 83$m^2$. $g^{-1}$.

Catalyseur D3.

On emploie les mêmes sels que ceux utilisés pour la préparation du catalyseur **D2** La solution de nitrates (volume= 6 litres; concentration 0,5 at.g./L,T= 75 °C et celle de carbonate de sodium (volume= 7,5 litres, concentration 1,2 at.g.Na/l,T= 75 °C), sont mises en réaction dans le même réacteur que celui

décrit pour la préparation du catalyseur A, opérant en continu, mais à 75° C; le pH varie entre 7,0 et 7,05 unités pH et le temps de séjour moyen est de 8 minutes.

Après 16 heures de mûrissement (temp. ambiante) dans ses eaux mères, le coprécipité est lavé en 3 fois par 45 litres (3 x 15 l) d'eau bipermutée. Le précipité lavé, homogène, est constitué d'une phase cristallisée; sa teneur en sodium résiduel est de 0,025% par rapport aux métaux.

Ce précipité est malaxé avec 0,05 litres d'une solution contenant 0,64 g de carbonate disodique (0,012 at.g.Na) puis il est mûri 12 h à 35° C et finalement séché par atomisation puis activé thermiquement pendant 3 heures à 350° C, pastillé avec ajout de 1,5% en poids d'acide stéarique et réactivation pendant 2 heures à 300° C. On obtient 165 g de pastilles (3 X 3 mm) de densité de remplissage 1,3 $kg.l^{-1}$ et de surface spécifique $106,5 m^2. g^{-1}$.

Catalyseur D4.

On reproduit la préparation du catalyseur D3, à l'exception de l'addition d'alcalin et du mûrissement avant sèchage par atomisation. Le produit lavé est donc directement séché par atomisation, puis activé thermiquement pendant 3 heures à 350° C.

On obtient ainsi 120,5 g d'oxydes sous forme d'une poudre légère, (densité apparente 0,1 $kg\ l^{-1}$). Cette poudre est malaxée dans un appareil WARIG-BLENDER avec 0,2 litre de solution contenant 0,64 g de carbonate disodique (0,012 at.g. Na) puis séchés en 0,1 h dans un four micro-ondes et finalement activés, mis en forme et réactivés comme décrit ci-avant pour la préparation du catalyseur D3.

On obtient 153 g de pastilles (3 X 3 mm) de densité de remplissage 1,23 $kg.l^{-1}$ et de surface spécifique 76 $m^2.g^{-1}$.

Catalyseur E.

Ce catalyseur est préparé dans les mêmes conditions opératoires que celles décrites pour la préparation du catalyseur B; lanthane et praséodyme sont employés sous forme de nitrates et remplacent le cérium , le zirconium est employé sous forme de nitrate de zirconyle dihydraté. On prépare ainsi 226,8g d'oxydes avant alcalinisation.

L'alcalinisation effectuée au carbonate dipotassique aqueux est suivie d'un mûrissement hydrothermique en récipient hermétiquement fermé (T= 60° C t= 4 heures), puis le produit est séché, activé, pastillé et réactivé comme décrit ci-avant pour la préparation du catalyseur B.

On obtient 208 g de pastilles (3,5 X 4 mm) de densité de remplissage 1,23 $kg.l^{-1}$.

Catalyseur F.

On opère comme pour la préparation du catalyseur D4 jusqu'à l'activation thermique (3 heures à 350° C) 150 g d'oxides sous forme de produit activé sont mis en contact avec 0,6 g de palladium sous forme d'acétate de palladium et 0,3 g de rhodium sous forme d'acétylacétonate de rhodium III en solution alcoolique (180 ml d'éthanol). Après sèchage sous azote à 150° C et recalcination pendant 3 heures à 350° C, le catalyseur est ensuite alcalinisé séché, activé, mis en forme et réactivé comme décrit dans la préparation du catalyseur D4.

On obtient 117 g de pastilles (3 X 3 mm) de densité de remplissage 1,35 $kg\ l^{-1}$ et de surface 70 $m^2. g^{-1}$. Ce catalyseur contient 0,4% Pd et 0,2% Rh par rapport aux oxydes, soit 0,5% Pd et 0,25% Rh par rapport aux métaux.

Catalyseur G. (comparatif).

On dissout dans l'eau 241,6 g de nitrate cuivrique trihydraté (1 at. g. Cu) 145,52 g de nitrate de cobalt hexahydraté (0,5 at.g.Co) 400,9 g de nitrate de zirconyle dihydraté (1,5 at.g.Zr) en présence de 0,3 litre d'acide nitrique pur (d= 1,38); la solution (0,5 at.g/l) est étendue à 6 litres.

On dissout séparément 640 g de carbonate disodique anhydre dans 10 litres d'eau (solution 1,2 at.g. Na/litre).

Les deux solutions portées à 75° C sont ajoutées simultanément dans un réacteur de 2,0 litres, contenant 2 litres d'eau portés à 75° C; ce réacteur est muni d'une surverse, les débits des deux solutions sont régulés par le pH, mesuré dans la zone de turbulence de la turbine d'agitation (turbine STARO). La durée de précipitation est de 1h 30 minutes et le temps de séjour moyen, de 12,4 minutes; le pH varie entre 6,95 et 7,04 unités pH.

Le précipité, mûri 20 heures à 40°C en présence de ses eaux mères, est ensuite lavé par 36 litres d'eau bipermutée (3 lavages par 12 litres). Il contient 28% en poids d'oxydes et 0,08% en poids Na par rapport aux métaux.

Le précipité lavé est placé dans un malaxeur WERNER et sous agitation, mélangé avec 0,1 litre d'une solution contenant 3,68 g de carbonate dipotassique anhydre (0,054 at.g K). La pâte est mûrie pendant 10 heures à 40°C puis séchée par atomisation; la température moyenne est de 120 - 140°C; la durée du sèchage est de 1 seconde.

Le produit séché obtenu, qui contient 77% en poids d'oxydes, est activé thermiquement sous air à 350°C pendant 4 heures, mélangé avec 2% de graphite naturel puis pastillé en cylindres de diamètre et de hauteur 4 mm.

Après activation thermique (300°C, 2 heures, sous azote) on obtient 200 g de pastilles dont la densité de remplissage est de 1,2 kg/$l^{-1}$.

Catalyseur H (comparatif )

Le catalyseur H est préparé en suivant le mode opératoire décrit pour la préparation du catalyseur A avec des quantités différentes des mêmes sels que ceux employés pour préparer le catalyseur A. L'alcalinisation est faite avec du carbonate dipotassique uniquement.

Catalyseur I (comparatif)

Le catalyseur I est préparé de même en suivant le mode opératoire décrit pour la préparation du catalyseur D4 avec des quantités différentes des mêmes sels que ceux utilisés pour préparer le catalyseur D4. L'alcalinisation est faite avec du carbonate dipotassique, en lieu et place du carbonate disodique utilisé dans la préparation du catalyseur D4.

Catalyseur J (comparatif)

On prépare 5 litres d'une solution contenant :
244,00 g de nitrate de cuivre trihydraté (1,01 Cu)
7,28 g de nitrate de cobalt hexahydraté (0,025 Co)
38,67 g de nitrate de zinc hexahydraté (0,13 Zn).

Cette solution contient 0,233 at.g/l de métaux. On dissout séparément 170 g de carbonate dissodique dans 7 l d'eau bipermutée (solution 0,46 at.g.Na/litre).

Les 2 solutions portées à 80°C sont ajoutées simultanément dans un réacteur de 2,0 litres contenant 1,5 litre d'eau porté à 80°C et muni d'une surverse. Les débits des deux solutions sont régulés par le pH qui, mesuré dans la zone de turbulence de la turbine (turbine STARO) est maintenu entre 6,95 et 7,05 unités pH.

Le précipité mûri 20 heures à 40°C en présence de ses eaux mères, est ensuite lavé par 30 litres d'eau permutée (3 lavages par 10 litres d'eau), il contient 30% en poids d'oxydes et 0,10% en poids de sodium par rapport aux métaux.

L'ajout de l'alcalin est effectué par malaxage (mélangeur HOBART) des 320 g de précipité lavé avec 1,85 g de carbonate dipotassique (0,027 at.g.K) et 1,85 g de carbonate dirubidique (0,016 at.g.Rb) dans 0,05 litre d'eau. Le précité est malaxé 3 heures puis mûri 10 heures à 25°C avant séchage par atomisation à la température moyenne de 110°C, temps de séjour moyen t = 1 seconde. Le produit séché obtenu contient 75% d'oxydes, il est activé sous air, pastillé et activé sous azote dans les conditions décrites pour la préparation du catalyseur A.

On obtient 80 g de pastilles de densité de remplissage 1,10 kg.$l^{-1}$.

Tests des catalyseurs :

Les catalyseurs des exemples A à J ont été testés dans une unité pilote fonctionnant en continu et opérant sur 50 ml de catalyseur. L'unité est alimentée à partir d'un mélange synthétique de gaz dont les proportions relatives en $H_2$-CO-$CO_2$ sont très proches de celles mesurées en unité industrielle; elle comprend un recyclage partiel des gaz non transformés.

Les exemples d'utilisation n°1 à 23 des catalyseurs A à J décrits au tableau 3 illustrent les performances et la stabilité améliorées des catalyseurs de l'invention (A à F). Les performances à 2000 heures des catalyseurs A à F sont décrites au tableau 4 pour les exemples d'utilisation 1, 3, 9 à 12, 14, 16

et 17 et pour les catalyseurs de comparaison G et H exemples 19 et 20.

Les performances sont définies comme suit :

- productivité massique en alcools r : c'est le nombre de grammes d'alcools obtenus par heure, ramené au poids (en grammes) de catalyseur chargé, elle s'exprime en heures$^{-1}$ ($h^{-1}$).
- productivité volumique en alcools p : c'est le nombre de gramme d'alcools obtenus par heure, ramené au volume (en centimètres cubes) de catalyseur chargé; elle s'exprime en $g.cm^{-3}h^{-1}$.
- sélectivité poids en alcools supérieurs $C_2^+$ OH %, c'est le rapport pondéral 100 x poids d'alcools $C_2^{+5}$ OH / poids total d'alcools formé, (S $C_2^+$OH).
- sélectivité de la transformation du CO et du $CO_2$ en alcools $S_A$; soit $C_1OH$, $C_2OH$, $C_3OH$, $C_4OH$.... $C_n$ OH le nombre de molécules-grammes formées pour chaque alcool, on en déduit que $N_C = C_1OH + 2\ C_2OH + 3\ C_3OH + 4\ C_4OH + ... + nC_nOH$ est le nombre de molécules grammes de CO transformées en alcools.

La sélectivité $S_A$ s'exprime donc par :

$$S_A = 100\ X \frac{Nc}{\text{mol.g } (CO + CO_2)_{\text{entrée}} - \text{mol.g } (CO + CO_2)_{\text{sortie}}}$$

(les sous-produits de la réaction sont le méthane, les hydrocarbures $C_2^+$ ainsi que certains composés oxygénés tels que les aldéhydes, les esters et les cétones, présents à l'état de traces). Les conditions de réduction préalable des catalyseurs sont détaillées tableau 2 et reportée tableaux 3 et 4.

Les exemples 1 à 18 illustrent les performances et la stabilité améliorée des catalyseurs A à F.

Les exemples 2 et 4 comparés respectivement aux exemples 3 et 5 montrent l'effet favorable de la pression partielle ($H_2 + CO + CO_2$) sur les performances.

Les exemples 5 à 8 montrent l'influence du rapport moyen H2/CO (molaire) sur les performances du catalyseur B. Bien que les productivités en alcools r et p diminuent légèrement lorsque H2/CO diminue, cette diminution permet d'augmenter sensiblement la sélectivité poids en alcools supérieurs ($SC_2$ + OH%) sans diminution appréciable de la sélectivité en alcools, $S_A$, qui reste très bonne.

L'exemple 13 (comparé à l'exemple 12 ) montre qu'à concentration en $CO_2$ plus élevée (8,9 % au lieu de 1,3%) le catalyseur D4 devient légèrement moins actif et très légèrement moins performant ($SC_2$ + OH, $S_A$) ; dans le même temps, la proportion d'eau dans les alcools produits, 1,5% poids, devient égale à 10,8% poids, ce qui implique le fractionnement ultérieur.

L'exemple 17 (comparé à 13) montre l'effet promoteur des métaux additionnels palladium et rhodium, sur l'activité (température de réaction plus basse) et sur les performances.

Les exemples 19 à 23 décrivent les résultats obtenus avec les catalyseurs de comparaison G , H , I , et J . Lors de la mise sous gaz de synthèse, une importante réaction de méthanation s'est produite avec le catalyseur I; l'élévation de température dans le lit (T= 250°C avant ajout du gaz de synthèse) a été d'environ 150°C (température maximum = 400°C ) ce qui explique les mauvaises performances obtenues, après une relative stabilisation des températures.

Tableau 1

| Catalyseurs (formules) | % poids de métal par rapport Σ nds des métaux | | | | | | | rapports atomiques | | | densité de remplissage |
|---|---|---|---|---|---|---|---|---|---|---|---|
| | Cu | Co | Zn | Zr | A | M | N | Cu/Co | $\frac{Zn+Zr}{Co}$ | $\frac{Zn}{Zn+Zr}$ | |
| A $Cu_{0,8}\ Co_{0,6}\ Zr_{1,0}\ Zn_{0,5}\ Na_{0,046}\ K_{0,016}$ | 24 | 16,7 | 15,44 | 43,07 | 0,8 | 0 | 0 | 1,33 | 2,5 | 0,323 | 1,12 |
| $A_1$ $Cu_{0,7}\ Co_{0,5}\ Zn_{1,7}\ Zr_{0,05}\ Na_{0,012}$ | 23,40 | 15,50 | 58,50 | 2,40 | 0,15 | 0 | 0 | 1,4 | 3,5 | 0,97 | 1,15 |
| B $Cu_{1,7}\ Co_{0,8}\ Zn_{1,0}\ Ce_{0,2}\ K_{0,045}\ Rb_{0,06}$ | 43,1 | 18,8 | 26,06 | 0 | 0,91 | 11,17 | 0 | 2,12 | 1,25 | 1 | 1,18 |
| C $Cu_1\ Co_{0,4}\ Zr_{0,6}\ Zn_{1,4}\ Nd_{0,2}\ Na_{0,169}\ Cs_{0,0045}$ | 23,8 | 8,84 | 34,32 | 20,52 | 1,69 | 10,82 | 0 | 2,5 | 5 | 0,7 | 1,05 |
| D1 $Cu_{1,3}\ Co_{0,5}\ Zn_{1,2}\ Na_{0,012}$ | 43,3 | 15,44 | 41,12 | 0 | 0,15 | 0 | 0 | 2,6 | 2,4 | 1 | 1,35 |
| D2 $Cu_{1,3}\ Co_{0,5}\ Zn_{1,2}\ Na_{0,012}$ | 43,3 | 15,44 | 41,12 | 0 | 0,15 | 0 | 0 | 2,6 | 2,4 | 1 | 1,30 |
| D3 $Cu_{1,3}\ Co_{0,5}\ Zn_{1,2}\ Na_{0,012}$ | 43,3 | 15,44 | 41,12 | 0 | 0,15 | 0 | 0 | 2,6 | 2,4 | 1 | 1,30 |
| D4 $Cu_{1,3}\ Co_{0,5}\ Zn_{1,2}\ Na_{0,012}$ | 43,3 | 15,44 | 41,12 | 0 | 0,15 | 0 | 0 | 2,6 | 2,4 | 1 | 1,23 |
| E $Cu_{0,9}\ Co_{0,45}\ Zr_{0,1}\ Zn_{1,1}\ La_{0,05}\ Pr_{0,07}\ K_{0,03}$ | 31,3 | 14,51 | 39,36 | 4,99 | 0,64 | 9,2 | 0 | 2 | 2,67 | 0,916 | 1,23 |
| F $Cu_{1,3}\ Co_{0,5}\ Zn_{1,2}\ Na_{0,012}\ Pd_{0,09}\ Rh_{0,005}$ | 43,0 | 15,33 | 40,8 | 0 | 0,15 | 0 | 0,75 | 2,6 | 2,4 | 1 | 1,35 |
| G * $Cu_1\ Co_{0,5}\ Zr_{1,5}\ K_{0,054}$ | 27,4 | 12,7 | 0 | 59,0 | 0,91 | 0 | 0 | 2 | 3 | 0 | 1,2 |
| H * $Cu_1\ Co_{0,5}\ Zr_{1,5}\ Zn_{0,625}\ K_{0,054}$ | 23,29 | 10,80 | 14,98 | 50,15 | 0,77 | 0 | 0 | 2 | 4,25 | 0,04 | 1,17 |
| I * $Cu_{0,8}\ Co_{0,84}\ Zn_{1,17}\ K_{0,11}$ | 28,07 | 27,33 | 42,33 | 0 | 2,[illegible] | 0 | 0 | 0,95 | 1,39 | 1 | 1,04 |
| J * $Cu_{1,01}\ Co_{0,025}\ Zn_{0,13}\ K_{0,027}\ Pb_{0,016}$ | 86,55 | 1,99 | 11,46 | 0 | 2,1[illegible] | 0 | 0 | 40,4 | 5,2 | 1 | 1,10 |

(*) Catalyseurs de comparaisor

Tableau 2

Conditions de réduction préalable des catalyseurs (Tests décrits aux tableaux 3 et 4)

Mode de réduction

(a) - 2% hydrogène dans l'azote
- pression atmosphérique
- vitesse volumétrique horaire : 1500 $h^{-1}$
- paliers de 24 h à 160 - 190 - 210°C
8 h à 240°C

(b) conditions (a) mais avec
pression totale = 0,5 MPa
1% $H_2$ dans l'azote
et vitesse volumétrique horaire : 1000 $h^{-1}$

(c) condition (a) mais avec
pression totale = 0,5 MPa
1% $H_2$ dans l'azote
24 h à 160 et 190°C
8 h à 210°C
12 h à 240°C

(d) conditions (c) avec un palier supplémentaire de 10 h à 270°C

(e) conditions (c) avec deux paliers supplémentaires et une modification
8 h à 240°C
8 h à 270°C
10 h à 300°C

**TABLEAU 3**

| Ex. | Cat. | Red. | performances à t heures | VVH $h^{-1}$ | $CO_2$ vol.% | $H_2/CO$ mol. | P | T°C | r | p | $S_{C_2+OH}$% | $S_A$% |
|---|---|---|---|---|---|---|---|---|---|---|---|---|
| 1 | A | a | 145 | 3000 | 0,8 | 1,7 | 6 | 290 | 0,11 | 0,12 | 44 | 77 |
| 2 | A | a | 500 | 3000 | 0,8 | 1,7 | 10 | 290 | 0,16 | 0,179 | 41 | 78 |
| 3 | B | e | 145 | 10000 | 1,1 | 2 | 6 | 280 | 0,11 | 0,13 | 46 | 75 |
| 4 | B | e | 800 | 10000 | 1,1 | 2 | 10 | 290 | 0,195 | 0,23 | 43 | 76 |
| 5 | B | e | 900 | 3000 | 1,0 | 2,2 | 8 | 290 | 0,13 | 0,153 | 40 | 76 |
| 6 | B | e | 1050 | 3000 | 0,9 | 1,5 | 8 | 290 | 0,115 | 0,136 | 45 | 75 |
| 7 | B | e | 1120 | 3000 | 0,8 | 1,0 | 8 | 290 | 0,10 | 0,118 | 56,8 | 69 |
| 8 | B | e | 1210 | 3000 | 1,0 | 2,2 | 8 | 290 | 0,125 | 0,148 | 41 | 77 |
| 9 | $D_1$ | c | 145 | 6000 | 1,3 | 1,6 | 6 | 290 | 0,06 | 0,081 | 42 | 74 |
| 10 | $D_2$ | c | 145 | 6000 | 1,3 | 1,6 | 6 | 290 | 0,09 | 0,117 | 36 | 78 |
| 11 | $D_3$ | c | 145 | 6000 | 1,3 | 1,6 | 6 | 290 | 0,105 | 0,136 | 38 | 80 |
| 12 | $D_4$ | c | 145 | 6000 | 1,3 | 1,6 | 6 | 290 | 0,095 | 0,117 | 40 | 75,5 |
| 13 | $D_4$ | c | 300 | 6000 | 8,9 | 1,6 | 6 | 290 | 0,080 | 0,098 | 38 | 73,8 |
| 14 | C | b | 145 | 4000 | 1 | 1,5 | 8 | 295 | 0,120 | 0,126 | 46 | 76 |
| 15 | C | b | 145 | 10000 | 1 | 1,5 | 19 | 400 | 1,10 | 1,21 | 50 | 68,5 |
| 16 | E | d | 145 | 8000 | 0,8 | 1,5 | 6 | 290 | 0,10 | 0,123 | 39,2 | 79 |
| 17 | F | c | 145 | 6000 | 1,3 | 1,6 | 6 | 280 | 0,105 | 0,142 | 44 | 76 |
| 18 | A1 | a | 145 | 8000 | 0,8 | 1,7 | 8 | 290 | 0,12 | 0,14 | 42 | 79 |
| 19 | G* | b | 145 | 5000 | 1,0 | 2 | 8 | 295 | 0,08 | 0,096 | 34 | 69 |
| 20 | H* | b | 145 | 6000 | 1 | 2 | 8 | 300 | 0,09 | 0,105 | 36 | 67,5 |
| 21 | I* | e | 72 | 6000 | 1,2 | 2 | 8 | 280 | 0,02 | 0,02 | 49 | 46 |
| 22 | J* | e | 145 | 10000 | 1,1 | 2 | 6 | 280 | 0,03 | 0,04 | 14 | 75 |
| 23 | J* | e | 145 | 10000 | 1,1 | 2 | 18 | 400 | 0,65 | 0,715 | 32 | 62 |

(*) catalyseurs de comparaison

Ex = Exemple, Cat. = Catalyseur, red = pr... dure de réduction, P = pression part... e de ($H_2 + CO + CO_2$) en MPa

TABLEAU 4

| Performances à t= 2000 h | | | | | | | | | | | |
|---|---|---|---|---|---|---|---|---|---|---|---|
| Ex. | Cat. | red. | VVH $h^{-1}$ | $CO_2$ vol% | $H_2/CO$ mol. | P | T°C | r | P | $S_{C_2}$+ OH% | $S_A$% |
| 1 | A | a | 3000 | 0,8 | 1,7 | 8 | 295 | 0,12 | 0,134 | 45 | 76 |
| 3 | B | e | 10000 | 1,1 | 2 | 6 | 292 | 0,10 | 0,118 | 42 | 78 |
| 9 | $D_1$ | c | 6000 | 1,3 | 1,6 | 8 | 290 | 0,06 | 0,081 | 40 | 75,5 |
| 10 | $D_2$ | c | 6000 | 1,3 | 1,6 | 8 | 290 | 0,085 | 0,110 | 35 | 78,5 |
| 11 | $D_3$ | c | 6000 | 1,3 | 1,1 | 8 | 290 | 0,110 | 0,143 | 37 | 80 |
| 12 | $D_4$ | c | 6000 | 1,3 | 1,6 | 8 | 290 | 0,09 | 0,111 | 38 | 76 |
| 14 | C | b | 4000 | 1 | 1,5 | 9 | 295 | 0,13 | 0,137 | 44 | 77 |
| 16 | E | d | 8000 | 0,8 | 1,5 | 6 | 305 | 0,105 | 0,129 | 38 | 80 |
| 17 | F | c | 6000 | 1,3 | 1,6 | 8 | 285 | 0,10 | 0,135 | 47 | 75 |
| 19 | G * | b | 4000 | 1 | 2 | 10 | 305 | 0,07 | 0,084 | 31 | 58,5 |
| 20 | H * | b | 4000 | 1 | 2 | 10 | 300 | 0,06 | 0,07 | 38 | 64 |

Ex = exemple, Cat. = Catalyseur, red = procédure de réduction
P = pression partielle de ($H_2$ + CO + $CO_2$) en MPa
(*) catalyseurs de comparaison

## Revendications

**1.** Procédé de fabrication d'alcools primaires saturés par réaction d'oxydes de carbone (CO, $CO_2$) avec l'hydrogène en présence d'un catalyseur formé essentiellement des éléments suivants : le cuivre, le cobalt, le zinc, au moins un métal A choisi dans le groupe formé par les métaux alcalins et les métaux alcalinoterreux et éventuellement le zirconium et/ou au moins un métal M choisi dans le groupe formé par le scandium, l'yttrium et les métaux des terres rares de numéros atomiques 57 à 71 inclus et/ou au moins un métal N choisi dans le groupe formé par les métaux nobles du groupe VIII de la classification périodique des éléments, ledit catalyseur étant essentiellement exempt d'aluminium, de chrome, de fer, de vanadium et de manganèse, c'est-à-dire qu'il ne contient pas ces métaux hormis à l'état d'impuretés, c'est-à-dire habituellement moins de 0,1 % en poids, éventuellement apportées par les réactifs de départ et/ou les appareillages utilisés lors de sa préparation, et de préférence en quantité non décelable, le procédé étant caractérisé en ce que :

a/ les proportions en poids de chaque élément métallique rapportées au poids total des métaux présents sont de :

| | |
|---|---|
| cuivre | 15-55% |
| cobalt | 5-25% |
| zinc | 15-70% |
| métal A | 0,01-5% |
| zirconium | 0-55% |
| métal M | 0-20% |
| métal N | 0-1% |

b/ la somme des pourcentages en poids du zinc et du zirconium par rapport au poids total des métaux est de 15 à 70%,
c/ les rapports atomiques entre ces métaux sont de :
Cu/Co = 0,2: 1 à 5 : 1
Zn/(Zn + Zr) = 0,05 : 1 à 1 : 1
(Zn + Zr)/Co = 0,5 : 1 à 8 : 1.

**2.** Procédé selon la revendication 1 dans lequel

a/ les proportions en poids de chaque élément métallique rapportées au poids total des métaux présents sont de :

| | |
|---|---|
| cuivre | 15-55% |
| cobalt | 5-25% |
| zinc | 15-70% |
| zirconium | 1-45% |
| métal A | 0,01-5% |
| métal M | 0-20% |
| métal N | 0-1% |

b/ la somme des pourcentages en poids du zinc et du zirconium par rapport au poids total des métaux est de 20 à 65%,
c/ les rapports atomiques entre ces métaux sont de :
Cu/Co = 0,5: 1 à 3,5 : 1
Zn/(Zn + Zr) = 0,1 : 1 à 0,98 : 1
(Zn + Zr)/Co = 1 : 1 à 5,5 : 1.

**3.** Procédé selon la revendication 1 ou 2 dans lequel la proportion pondérale du métal M rapportée au poids total des métaux présents est de 0,1 à 15%.

**4.** Procédé selon l'une des revendications 1 à 3 dans lequel la proportion pondérale du métal N rapportée au poids total des métaux présents est de 0,01 à 0,8%.

**5.** Procédé selon l'une des revendications 1 à 4 dans lequel le catalyseur résulte du séchage et de l'activation thermique d'un précurseur hydraté, cristallisé au moins en partie, obtenu par coprécipitation entre une solution de sels solubles des métaux cuivre, cobalt, zinc et éventuellement du zirconium, éventuellement d'au moins un métal M et/ou N, de concentration globale au plus égale à 1 atome gramme (at.g) de métaux par litre et de préférence de 0,1 à 0,6 at.g. par litre, et une solution de carbonate et/ou de bicarbonate et/ou d'hydroxyde, de métaux alcalins, de préférence de sodium et/ou de potassium, et/ou d'ammonium, de concentration globale au plus égale à 2 at.g. de métaux alcalins et/ou $NH^{+}_{4}$ par litre et de préférence de 0,2 à 1,2 at.g. par litre, la réaction de coprécipitation étant menée à un pH de 7 ± 1 unité pH, à une température d'au moins 50° C et de préférence d'au moins 70° C et avec un temps de résidence dans le milieu réactionnel d'au moins 2 minutes et de préférence d'au moins 5 minutes, le coprécipité hydraté étant ensuite éventuellement mûri en présence de ses eaux mères, lavé jusqu'à réduire sa teneur en métaux alcalins (exprimée en poids de métaux alcalins par rapport aux métaux) à une valeur de 0,01 à 0,4% et de préférence de 0,05 à 0,2% poids, puis éventuellement mûri en présence de ses eaux de lavage, le mûrissement étant mené entre environ 15 et environ 250° C en présence d'eau liquide, pendant 15 minutes à 5 heures.

6. Procédé selon l'une des revendications 1 à 4 dans lequel le catalyseur résulte du sèchage et de l'activation thermique d'un précurseur hydraté dont la structure (déterminée par diffraction X) est amorphe, obtenu par coprécipitation entre une solution de sels solubles des métaux cuivre, cobalt, zinc et éventuellement du zirconium, éventuellement d'au moins un métal M et/ou N, de concentration globale supérieure ou égale à 1 at.g. de métaux par litre et une solution de carbonate et/ou de bicarbonate et/ou d'hydroxyde de métaux alcalins, de préférence de sodium et/ou de potassium, et/ou d'ammonium, de concentration globale au moins égale à 2 at.g. de métaux alcalins et/ou d'ammonium par litre, la réaction de coprécipitation étant menée entre 0 et 30° C à un pH de 7 ± 1 unité pH, à une température comprise entre 0 et environ 30° C et avec un temps de résidence dans le milieu réactionnel d'au plus 5 mn, le précurseur hydraté étant ensuite directement lavé jusqu'à réduire sa teneur en métaux alcalins (exprimée en poids de métaux alcalins par rapport aux métaux) à une valeur de 0,05 à 5% poids.

7. Procédé selon la revendication 5 ou 6, dans lequel les métaux alcalins sont introduits dans le catalyseur par ladite solution de carbonate et/ou de bicarbonate et/ou d'hydroxyde des dits métaux, le lavage étant mené de manière à laisser de 0,09 à 3,5% en poids de métaux alcalins, le précipité lavé étant ensuite séché, puis activé thermiquement à 250-600° C pendant au moins 0,5 heures.

8. Procédé selon l'une des revendications 5 ou 6 dans lequel au moins un métal alcalin et/ou alcalino-terreux est ajouté ultérieurement au précipité hydraté contenant au moins les métaux cuivre et cobalt et au moins une partie du zinc, ainsi qu'éventuellement du zirconium et/ou au moins un métal M et/ou N par mise en contact avec le précipité, filtration éventuelle, mûrissement éventuel, puis séchage, ledit séchage étant de préférence effectué par atomisation (spray-drying) à une température de 100 à 250° C en moins de 10 secondes.

9. Procédé selon l'une des revendications 1 à 8 dans lequel le catalyseur est soumis à une réduction avant utilisation, ladite réduction du catalyseur étant effectuée par mise en contact avec un mélange de gaz inerte et d'au moins un composé réducteur en rapport molaire gaz réducteur/ gaz inerte + gaz réducteur de 0,001 : 1 à 1 : 1, ledit gaz réducteur étant choisi dans le groupe formé par l'hydrogène, le monoxyde de carbone, les alcools et les aldéhydes en $C_1$ et $C_2$, la réduction étant menée entre 100 et 350° C, de préférence entre 130 et 320° C, sous une pression totale de 0,1 à 5 MPa et de préférence de 0,2 à 2 MPa et à une vitesse volumitrique horaire de $0,5.10^2$ à $1.10^4$ heures $^{-1}$ et de préférence de $10^2$ à $5.10^3$ heures $^{-1}$ (TPN).

10. Procédé selon l'une des revendications 1 à 9 dans lequel la réaction des oxydes de carbone avec l'hydrogène est mise en oeuvre à 250-350° C et de préférence 260-320° C, sous une pression partielle du mélange hydrogène et oxydes de carbone (CO, $CO_2$) de 2 à 15 MPa et de préférence de 5 à 12 MPa avec un rapport molaire moyen $H_2$/CO dans la zone réactionnelle de 0,1:1 à 4:1 et de préférence de 0,2:1 à 3,5:1, une VVH comprise entre 1000 et 40000$h^{-1}$ et de préférence comprise entre 2000 et 20000$h^{-1}$, la teneur moyenne en $CO_2$ dans la zone réactionnelle étant de 0 à 5% en volume et de préférence de 0,1 à 3% en volume.

11. Procédé selon l'une des revendications 1 à 10 dans lequel la réaction des oxydes de carbone avec l'hydrogène est mise en oeuvre en présence d'une phase liquide comprenant un ou plusieurs hydrocarbures ayant au moins 5 atomes de carbone par molécule.

12. Catalyseur pour la mise en oeuvre du procédé selon l'une quelconque des revendications 1 à 11 formé essentiellement des éléments suivants :

le cuivre, le cobalt, le zinc, au moins un métal A choisi dans le groupe formé par les métaux alcalins et les métaux alcalino-terreux et éventuellement le zirconium et/ou au moins un métal M choisi dans le groupe formé par le scandium, l'yttrium et les métaux des terres rares de numéros atomiques 57 à 71 inclus et/ou au moins un métal N choisi dans le groupe formé par les métaux nobles du groupe VIII de la classification périodique des éléments, ledit catalyseur étant essentiellement exempt d'aluminium, de chrome, de fer, de vanadium et de manganèse, c'est-à-dire qu'il ne contient pas ces métaux hormis à l'état d'impuretés, c'est-à-dire habituellement moins de 0,1 % en poids, éventuellement apportées par les réactifs de départ et/ou les appareillages utilisés lors de sa préparation, et de préférence en quantité non décelable, le catalyseur étant caractérisé en ce que :

a/ les proportions en poids de chaque élément métallique rapportées au poids total des métaux présents sont de :

| | |
|---|---|
| cuivre | 15-55% |
| cobalt | 5-25% |
| zinc | 15-70% |
| métal A | 0,01-5% |
| zirconium | 0-55% |
| métal M | 0-20% |
| métal N | 0-1% |

b/ la somme des pourcentages en poids du zinc et du zirconium par rapport au poids total des métaux est de 15 à 70% et de préférence de 20 à 65%
c/ les rapports atomiques entre ces métaux sont de :
Cu/Co = 0,2 : 1 à 5 : 1 et de préférence 0,5 : 1 à 3,5 : 1
Zn/(Zn + Zr) = 0,05 :1 à 1 : 1 et de préférence 0,1 : 1 à 0,98 : 1
(Zn + Zr)/Co = 0,5 : 1 à 8 : 1 et de préférence 1 : 1 à 5,5 : 1

**13.** Catalyseur selon la revendication 12 dans lequel la proportion pondérale du métal M, rapportée au poids total des métaux présents est de 0,1 à 15%.

**14.** Catalyseur selon la revendication 12 ou 13 dans lequel la proportion pondérale du métal N rapportée au poids total des métaux présents est de 0,01 à 0,8%.

**Claims**

**1.** A process for manufacturing saturated primary alcohols by reacting carbon oxides (CO, $CO_2$) with hydrogen in the presence of a catalyst essentially formed of the following elements : copper, cobalt, zinc, at least one metal A selected from the group formed of alkali metals and alkaline-earth metals and optionally zirconium and/or at least one metal M selected from the group formed of scandium, yttrium and rare-earth metals of atomic numbers from 57 to 71 inclusive and/or at least one metal N selected from the group formed of noble metals from group VIII of the periodic classification of elements, the said catalyst being essentially free of aluminium, chromium, iron, vanadium and manganese, i.e. none of these metals is present therein, except only as impurities which may originate from the starting reactants and/or the apparatus used during its preparation, usually in a proportion of less than 0.1% by weight and preferably in a non detectable amount, said process characterized in that :
a) the amount of each metal element, expressed in proportion to the total weight of the metals is :

| | |
|---|---|
| copper | 15 - 55 % |
| cobalt | 5 - 25 % |
| zinc | 15 - 70 % |
| metal A | 0.01 - 5 % |
| zirconium | 0 - 55 % |
| metal M | 0 - 20 % |
| metal N | 0 - 1 % |

b) The sum of the zinc and zirconium weights expressed in proportion to the total weight of the metals, is from 15 to 70 %.
c) The atomic ratios between these metals are :
Cu/Co = 0.2 :1 to 5:1
Zn/(Zn + Zr) = 0.05:1 to 1:1
(Zn + Zr)/Co = 0.5 :1 to 8:1

**2.** A process according to claim 1, wherein :
a) The amount by weight of each metal element, in proportion to the total weight of the metals, is :

| | |
|---|---|
| copper | 15 - 55 % |
| cobalt | 5 - 25 % |
| zinc | 15 - 70 % |
| zirconium | 1 - 45 % |
| metal A | 0.01 - 5 % |
| metal M | 0 - 20 % |
| metal N | 0 - 1 % |

b) The sum of the zinc and zirconium weights, expressed in proportion to the total weight of the metals, is from 20 to 65 %.

c) The atomic ratios between these metals are :

Cu/Co = 0.5:1 to 3.5 :1

Zn/(Zn + Zr) = 0.1:1 to 0.98:1

(Zn + Zr)/Co = 1 :1 to 5.5 :1

**3.** A process according to claims 1 or 2, wherein the amount of metal M, expressed in proportion to the total weight of the metals, is from 0.1 to 15 %.

**4.** A process according to one of claims 1 to 3, wherein the amount of metal N, expressed in proportion to the total weight of the metals, is from 0.01 to 0.8 %.

**5.** A process according to one of claims 1 to 4, wherein the catalyst results from the drying and thermal activation of a hydrated precursor, at least partly crystallized, obtained by coprecipitation between a solution of soluble salts of copper, cobalt, zinc and optionally zirconium, optionally at least one metal M and/or N, of total concentration at equal to 1 gram-atom (g.at.) of metals per liter and preferably from 0.1 to 0.6 g.at. per liter, and a solution of carbonate and/or bicarbonate and/or hydroxide of alkali metals, preferably of sodium and/or potassium and/or ammonium, of total concentration at most equal to 2 g.at. of alkali metals and/or $NH_4+$ per liter and preferably from 0.2 to 1.2 g.at. per liter, the coprecipitation reaction being conducted at a pH of 7± 1 pH units, at a temperature of a least 50° C and preferably at least 70° C and with a residence time in the reaction medium of at least 2 minutes and preferably at least 5 minutes, the hydrated coprecipitate being then optionally matured in the presence of its mother-liquors, washed until its alkali metal content (weight of alkali metals expressed in proportion to all the metals) be reduced to a value from 0.01 to 0.4 % and preferably from 0.05 to 0.2 %, then optionally matured in the presence of its mother-liquors, the maturation being conducted within the range of about 15 ° C to about 250° C in the presence of liquid water, for 15 minutes to 5 hours.

**6.** A process according to one of claims 1 to 4, wherein the catalyst results from the drying and thermal activation of a hydrated precursor, whose structure (determined by X-ray diffraction) is amorphous, obtained by coprecipitation between a solution of soluble salts of copper, cobalt, zinc and optionally zirconium, optionally at least one metal M and/or N, at a total concentration higher than or equal to 1 g.at. of metals per liter and a solution of carbonate and/or bicarbonate and/or hydroxide of alkali metals, preferably of sodium and/or potassium and/or ammonium, at a total concentration of at least 2 g.at. of alkali metals and/or ammonium per liter, the coprecipitation reaction being conducted at a pH of 7± 1 pH units, at a temperature ranging from 0 to about 30° C and with a residence time in the reaction medium of at most 5 mn, the hydrated precursor being there directly washed until its alkali metal content (expressed as the weight of alkali metals in proportion to all the metals) be reduced to a value from 0.05 to 5 % by weight.

**7.** A process according to claim 5 or 6, wherein the alkali metals are introduced in the catalyst through said solution of carbonate and/or bicarbonate and/or hydroxide of said metals, the washing step being conducted so as to keep from 0.09 to 3.5 % by weight of alkali metals, the washed precipitate being then dried, then thermally activated at 250 - 600° C for at least 0.5 hour.

**8.** A process according to one of claims 5 or 6, wherein at least one alkali and/or alkaline-earth metal is subsequently added to the hydrated precipitate containing at least copper and cobalt and at least a part of the zinc, as well as optionally zirconium and/or at least one metal M and/or N, by contact with the precipitate, optional filtration, optional maturation, then drying, said drying being preferably a spray-drying at a temperature of 100 to 250 °C for at least 10 seconds.

**9.** A process according to one of claims 1 to 8, wherein the catalyst is subjected to a reduction before use, said catalyst reduction being performed by contact with a mixture of inert gas with at least one reducing compound in a molar ratio "reducing gas/inert gas + reducing gas" from 0.001:1 to 1:1, said reducing gas being selected from the group formed of hydrogen, carbon monoxide, alcohols and $C_1$-$C_2$ aldehydes, the reduction being conducted between 100 and 350 °C, preferably between 130 and 320 °C, under a total pressure from 0.1 to 5 MPa, preferably from 0.2 to 2 MPa, at an hourly volume velocity from 50 to 10 000 $hours^{-1}$, preferably from 100 to 5 000 $hours^{-1}$ (NTP).

**10.** A process according to one of claims 1 to 9, wherein the reaction of carbon oxides with hydrogen is conducted at 250 - 350 °C, preferably 260 - 320 °C, under a partial pressure of the hydrogen and carbon oxides (CO, $CO_2$) mixture from 2 to 15 MPa and preferably from 5 to 12 MPa, with an average $H_2$/CO molar ratio in the reaction zone from 0.1:1 to 4:1 and preferably from 0.2:1 to 3.5:1, a VVH from 1 000 to 40 000 $h^{-1}$ and preferably from 2 000 to 20 000 $h^{-1}$, the average $CO_2$ content in the reaction zone being from 0 to 5 % by volume and preferably from 0.1 to 3 % by volume.

**11.** A process according to one of claims 1 to 10, wherein the reaction of carbon oxides with hydrogen is conducted in the presence of a liquid phase comprising one or more hydrocarbons having at least 5 carbon atoms per molecule.

**12.** A catalyst for carrying out the process according to any one of claims 1 to 11, essentially formed of the following elements: copper, cobalt, zinc, at least one metal A selected from the group formed of alkali metals and alkaline-earth metals and optionally zirconium and/or at least one metal M selected from the group formed of scandium, yttrium and rare-earth metals of atomic numbers from 57 to 71 inclusive and/or at least one metal N selected from the group formed by the noble metals of group VIII of the periodic classification of elements, the said catalyst being essentially free of aluminium, chromium, iron, vanadium and manganese, i.e. none of these metals is present therein, except only as impurities which may originate from the starting reactants and/or the apparatus used during its preparation, usually in a proportion of less than 0.1% by weight and preferably in a non detectable amount.

a) the amount by weight of each metal element, expressed in proportion to the total weight of the metals, being :

| | |
|---|---|
| copper | 15 - 55 % |
| cobalt | 5 - 25 % |
| zinc | 15 - 70 % |
| metal A | 0.01 - 5 % |
| zirconium | 0 - 55 % |
| metal M | 0 - 20 % |
| metal N | 0 - 1 % |

b) the sum of zinc and zirconium weights, expressed in proportion to the total weight of the metals, is from 15 to 70 % and preferably from 20 to 65 %.

c) the atomic ratios between these metals are :

Cu/Co = 0.2 :1 to 5:1 and preferably 0.5:1 to 3.5 :1
Zn/(Zn + Zr) = 0.05:1 to 1:1 and preferably 0.1:1 to 0.98:1
(Zn + Zr)/Co = 0.5 :1 to 8:1 and preferably 1 :1 to 5.5 :1

**13.** A catalyst according to claim 12, wherein the weight of metal M, expressed in proportion to the total weight of the metals, is from 0.1 to 15 %

**14.** A catalyst according to claims 12 or 13, wherein the weight of metal N, expressed in proportion to the total weight of the metals, is from 0.01 to 0.8 %.

**Patentansprüche**

1. Verfahren zur Herstellung von gesättigten primären Alkoholen durch Umsetzung von Kohlenstoffoxiden (CO, $CO_2$) mit Wasserstoff in Gegenwart eines Katalysators, der im wesentlichen aus den folgenden Elementen gebildet worden ist: Kupfer, Kobalt, Zink, mindestens einem Metall A, ausgewählt aus der Gruppe, bestehend aus den Alkalimetallen und den Erdalkalimetallen,und gegebenenfalls Zirkonium und/oder mindestens einem Metall M, ausgewählt aus der Gruppe, bestehend aus Scandium, Yttrium und den Metallen der Seltenen Erden mit den Atomzahlen 57 bis einschließlich 71, und/oder mindestens einem Metall N, ausgewählt aus der Gruppe, bestehend aus den Edelmetallen der Gruppe VIII des Periodischen Systems der Elemente, wobei der Katalysator im wesentlichen frei von Aluminium, Chrom, Eisen, Vanadin und Mangan ist, d.h. diese Metalle nicht enthält außer als Verunreinigungen, das heißt, in der Regel in einer Menge von weniger als 0,1 Gew.-%, die gegebenenfalls stammen aus den Ausgangsreagentien und/oder den bei ihrer Herstellung verwendeten Apparaturen, und vorzugsweise in einer nicht nachweisbaren Menge enthält, wobei das Verfahren dadurch gekennzeichnet ist, daß

(a) die Gewichtsverhältnisse jedes Metallelements, bezogen auf das Gesamtgewicht der vorhandenen Metalle, betragen:

| | |
|---|---|
| Kupfer | 15 - 55 % |
| Kobalt | 5 - 25 % |
| Zink | 15 - 70 % |
| Metall A | 0,01 - 5 % |
| Zirkonium | 0 - 55 % |
| Metall M | 0 - 20 % |
| Metall N | 0 - 1 % |

(b) die Summe der Gewichtsprozentsätze an Zink und Zirkonium, bezogen auf das Gesamtgewicht der Metalle, 15 - 70 % beträgt und
(c) die Atomverhältnisse zwischen diesen Metallen betragen:
Cu/Co = 0,2:1 bis 5:1
Zn/(Zn + Zr) = 0,05:1 bis 1:1
(Zn + Zr)/Co = 0,5:1 bis 8:1

2. Verfahren nach Anspruch 1, worin

(a) die Gewichtsverhältnisse jedes Metallelements, bezogen auf das Gesamtgewicht der vorhandenen Metalle, betragen:

| | |
|---|---|
| Kupfer | 15 - 55 % |
| Kobalt | 5 - 25 % |
| Zink | 15 - 70 % |
| Zirkonium | 1 - 45 % |
| Metall A | 0,01 - 5 % |
| Metall M | 0 - 20 % |
| Metall N | 0 - 1 % |

(b) die Summe der Gewichtsprozentsätze an Zink und Zirkonium, bezogen auf das Gesamtgewicht der Metalle, 20 - 65 % beträgt und
(c) die Atomverhältnisse zwischen diesen Metallen betragen:
Cu/Co = 0,5:1 bis 3,5:1
Zn/(Zn + Zr) = 0,1:1 bis 0,98:1
(Zn + Zr)/Co = 1:1 bis 5,5:1

3. Verfahren nach Anspruch 1 oder 2, worin das Gewichtsverhältnis des Metalls M, bezogen auf das Gesamtgewicht der vorhandenen Metalle, 0,1 bis 15 % beträgt.

4. Verfahren nach einem der Ansprüche 1 bis 3, worin das Gewichtsverhältnis des Metalls N, bezogen auf das Gesamtgewicht der vorhandenen Metalle, 0,01 bis 0,8 % beträgt.

5. Verfahren nach einem der Ansprüche 1 bis 4, worin der Katalysator erhalten wird durch Trocknen und thermisches Aktivieren eines hydratisierten, mindestens zum Teil kristallisierten Vorläufers, der erhalten wird duch Copräzipitation einer Lösung von löslichen Salzen der Metalle Kupfer, Kobalt, Zink und ggf. Zirkonium, ggf. mindestens eines Metalls M und/oder N in einer Gesamtkonzentration von höchstens 1g-Atom (g-at) Metalle pro Liter und vorzugsweise 0,1 bis 0,6 g-at pro Liter, und einer Lösung eines Carbonats und/oder Bicarbonats und/oder Hydroxids von Alkalimetallen, vorzugsweise von Natrium und/oder Kalium und/oder Ammonium in einer Gesamtkonzentration von höchstens 2 g-at der Alkalimetalle und/oder $NH_4^{+5}$ pro Liter und vorzugsweise von 0,2 bis 1,2 g-at pro Liter, wobei die Copräzipitationsreaktion bei einem pH-Wert von 7 ± 1, bei einer Temperatur von mindestens 50° C und vorzugsweise von mindestens 70° C und mit einer Verweilzeit in dem Reaktionsmilieu von mindestens 2 Minuten und vorzugsweise mindestens 5 Minuten durchgeführt wird, das hydratisierte Copräzipitat anschließend ggf. in Gegenwart seiner Mutterlaugen reifengelassen wird, gewaschen wird bis zur Herabsetzung seines Gehaltes an Alkalimetallen (ausgedrückt in Gew.-% Alkalimetalle, bezogen auf die Metalle) auf einen Wert von 0,01 bis 0,4 % und vorzugsweise von 0,05 bis 0,2 %, und dann gegebenenfalls in Gegenwart seiner Mutterlaugen reifengelassen wird, wobei die Reifung 15 Minuten bis 5 Stunden lang bei einer Temperatur zwischen etwa 15 und etwa 250° C in Gegenwart von flüssigem Wasser durchgeführt wird.

6. Verfahren nach einem der Ansprüche 1 bis 4, worin der Katalysator erhalten wird durch Trocknung und thermische Aktivierung eines hydratisierten Vorläufers, dessen Struktur (bestimmt durch Röntgenbeugung) amorph ist, der erhalten wurde durch Copräzipitation einer Lösung von löslichen Salzen der Metalle Kupfer, Kobalt, Zink und ggf. Zirkonium, ggf. mindestens eines Metalls M und/oder N, in einer Gesamtkonzentration von mindestens gleich 1 g-at der Metalle pro Liter und einer Lösung eines Carbonats und/oder Bicarbonats und/oder Hydroxids der Alkalimetalle, vorzugsweise von Natrium und/oder Kalium und/oder Ammonium in einer Gesamtkonzentration von mindestens 2 g-at Erdalkalimetalle und/oder Ammonium pro Liter, wobei die Copräzipitationsreaktion zwischen 0 und 30° C bei einem pH-Wert von 7 ± 1 bei einer Temperatur zwischen 0 und etwa 30° C und bei einer Verweilzeit in dem Reaktionsmilieu von höchstens 5 min durchgeführt wird, und der hydratisierte Vorläufer anschließend direkt gewaschen wird bis zur Herabsetzung seines Gehaltes an Alkalimetallen (ausgedrückt in Gew.-% Alkalimetalle, bezogen auf die Metalle) auf einen Wert von 0,05 bis 5 Gew.-%.

7. Verfahren nach Anspruch 5 oder 6, worin die Alkalimetalle in den Katalysator eingeführt werden durch die genannte Lösung eines Carbonats und/oder Bicarbonats und/oder Hydroxids der genannten Metalle, wobei das Waschen in der Weise durchgeführt wird, daß 0,09 bis 3,5 Gew.-% Alkalimetalle zurückbleiben, das Präzipitat anschließend getrocknet wird und dann mindestens 0,5 Stunden lang bei 250 bis 600° C thermisch aktiviert wird.

8. Verfahren nach einem der Ansprüche 5 und 6, worin mindestens ein Alkalimetall und/oder ein Erdalkalimetall dem hydratisierten Präzipitat schließlich zugesetzt wird, das mindestens die Metalle Kupfer und Kobalt und mindestens einen Teil des Zinks sowie ggf. des Zirkoniums und/oder mindestens eines Metalls M und/oder N enthält, indem man es mit dem Präzipitat in Kontakt bringt, ggf. filtriert, ggf. reifen läßt, dann trocknet, wobei die Trocknung vorzugsweise durch Zerstäubung (Sprühtrocknung) bei einer Temperatur von 100 bis 250° C in einem Zeitraum von weniger als 10 Sekunden durchgeführt wird.

9. Verfahren nach einem der Ansprüche 1 bis 8, worin der Katalysator vor seiner Verwendung einer Reduktion unterworfen wird, wobei die Reduktion des Katalysators durchgeführt wird durch Kontaktieren desselben mit einem Gemisch aus einem inerten Gas und mindestens einer reduzierenden Verbindung in einem Molverhältnis von Gas-Reduktionsmittel/Inertgas + Gas-Reduktionsmittel von 0,001:1 bis 1:1, wobei das Gas-Reduktionsmittel ausgewählt wird aus der Gruppe, die besteht aus Wasserstoff, Kohlenmonoxid, Alkoholen und Aldehyden mit 1 und 2 Kohlenstoffatomen, die Reduktion zwischen 100 und 350° C, vorzugsweise zwischen 130 und 320° C, unter einem Gesamtdruck von 0,1 bis 5 MPa, vorzugsweise von 0,2 bis 2 MPa, und mit einer stündlichen Raumgeschwindigkeit von 0,5 x $10^2$ bis 1 x $10^4$ Stunden$^{-1}$, vorzugsweise von $10^2$ bis 5 x $10^3$ Stunden$^{-1}$,(TPN) durchgeführt wird.

10. Verfahren nach einem der Ansprüche 1 bis 9, worin die Umsetzung der Kohlenstoffoxide mit Wasserstoff bei 250 bis 350° C, vorzugsweise 260 bis 320° C, unter einem Partialdruck des Gemisches aus Wasserstoff und Kohlenstoffoxiden (CO, $CO_2$) von 2 bis 15 MPa, vorzugsweise von 5 bis 12 MPa, mit

einem mittleren Molverhältnis $H_2/CO$ in der Reaktionszone von 0,1:1 bis 4:1, vorzugsweise von 0,2:1 bis 3,5:1, bei einer VVH zwischen 1 000 und 40 000 $h^{-1}$, vorzugsweise zwischen 2 000 und 20 000 $h^{-1}$ durchgeführt wird, wobei der mittlere Gehalt an $CO_2$ in der Reaktionszone 0 bis 5 Vol.-%, vorzugsweise 0,1 bis 3 Vol.-%,beträgt.

**11.** Verfahren nach einem der Ansprüche 1 bis 10, worin die Umsetzung der Kohlenstoffoxide mit Wasserstoff in Gegenwart einer flüssigen Phase durchgeführt wird, die ein oder mehr Kohlenwasserstoffe mit mindestens 5 Kohlenstoffatomen pro Molekül enthält.

**12.** Katalysator zur Durchführung des Verfahrens nach einem der Ansprüche 1 bis 11, der im wesentlichen aus den folgenden Elementen gebildet ist:

Kupfer, Kobalt, Zink, mindestens einem Metall A, ausgewählt aus der Gruppe, bestehend aus den Alkalimetallen und den Erdalkalimetallen, und ggf. Zirkonium und/oder mindestens einem Metall M, ausgewählt aus der Gruppe, bestehend aus Scandium, Yttrium und den Metallen der Seltenen Erden mit den Atomzahlen 57 bis einschließlich 71, und/oder mindestens einem Metall N, ausgewählt aus der Gruppe, bestehend aus den Edelmetallen der Gruppe VIII des Periodischen Systems der Elemente, wobei der Katalysator im wesentlichen frei von Aluminium, Chrom, Eisen, Vanadin und Mangan ist, d.h. diese Metalle nicht enthält außer als Verunreinigungen, d.h. in der Regel in einer Menge von weniger als 0,1 Gew.-%, die ggf. stammen aus den Ausgangsreagentien und/oder den für ihre Herstellung verwendeten Apparaturen, und vorzugsweise in einer nicht nachweisbaren Menge enthält, wobei der Katalysator dadurch gekennzeichnet ist, daß

(a) die Gewichtsverhältnisse jedes Metallelements, bezogen auf das Gesamtgewicht der vorhandenen Metalle, betragen:

| | |
|---|---|
| Kupfer | 15 - 55 % |
| Kobalt | 5 - 25 % |
| Zink | 15 - 70 % |
| Metall A | 0,01 - 5 % |
| Zirkonium | 0 - 55 % |
| Metall M | 0 - 20 % |
| Metall N | 0 - 1 % |

(b) die Summe der Gewichtsprozentsätze an Zink und Zirkonium, bezogen auf das Gesamtgewicht der Metalle, 15 - 70 %, vorzugsweise 20 - 65 %, beträgt, und
(c) die Atomverhältnisse zwischen diesen Metallen betragen:
Cu/Co = 0,2 : 1 bis 5 : 1, vorzugsweise 0,5 : 1 bis 3,5 : 1
Zn/(Zn + Zr) = 0,05 : 1 bis 1 : 1, vorzugsweise 0,1 : 1 bis 0,98 : 1
(Zn + Zr)/Co = 0,5 : 1 bis 8 : 1, vorzugsweise 1 : 1 bis 5,5 : 1

**13.** Katalysator nach Anspruch 12, worin das Gewichtsverhältnis des Metalls M, bezogen auf das Gesamtgewicht der vorhandenen Metalle, 0,1 bis 15 % beträgt.

**14.** Katalysator nach Anspruch 12 oder 13, worin das Gewichtsverhältnis des Metalls N, bezogen auf das Gesamtgewicht der vorhandenen Metalle, 0,01 bis 0,8 % beträgt.